# EUROPEAN PATENT APPLICATION

(11) **EP 1 136 547 A2**
(43) Date of publication of application: **26.09.2001**
(21) Application number: 01302634.9
(22) Date of filing: 21.03.2001
(51) Int. Cl.: C12N 9/64, C12N 5/10, C12P 21/02, C07K 16/40, C12N 15/52, C12N 15/74, C12N 15/79, A61K 38/48, C12Q 1/02, C12Q 1/68, G01N 33/53, G01N 33/68, A61P 19/00, A61P 11/00, A61P 25/00, A61P 35/00

(54) **Adamts polypeptides, nucleic acids encoding them, and uses thereof**

(30) Priority: 22.03.2000 US 191382 P
(71) Applicant: Pfizer Products Inc., Groton, Connecticut 06340 (US)
(72) Inventor: Buckbinder, Leonard, Pfizer Global Res. and Dev., Groton, Connecticut 06340 (US); Mitchell, Peter Geoffrey, Pfizer Global Res. & Dev, Groton, Connecticut 06340 (US); Schaefer, Jean Frances, Pfizer Global Res. & Dev., Groton, Connecticut 06340 (US); Walsh, Roderick Thomas, Pfizer Global Res. & Dev., Groton, Connecticut 06340 (US)
(74) Representative: Hayles, James Richard

(57) **Abstract**

The present invention relates to a member of the family of proteins known as ADAMTS proteins, the new member being designated ADAMTS-SI. The invention also relates to polynucleotides encoding ADAMTS-SI, antibodies to ADAMTS-SI, assays for studying the function of ADAMTS-SI, assays for determining agonists or antagonists of ADAMTS-SI, and to the use of ADAMTS-SI polypeptides or polynucleotides in diagnostic, biotherapeutic, or gene therapy methods.

SEQUENCE LISTING

<110> PFIZER PRODUCTS INC.

<120> ADAMTS POLYPEPTIDES, NUCLEIC ACIDS ENCODING THEM, AND
      USES THEREOF

<130> PC10850A

<140>
<141>

<160> 10

<170> PatentIn Ver. 2.1

<210> 1
<211> 5949
<212> DNA
<213> Human

<400> 1
ccggaattcc cgggtcgacc cacgcgtccg gccccccatt caagaagccg ctcagctatc 60
ccggccagca cagggcgccc ggcgcgcctc ggagcgcaag ttcctcgcct tctcctgccc 120
gctcgctggg cattatgcgg ccaagcagcc gagccccagt cctcctcctc ctcctgctcc 180
tccggctcct cctgcggccc gagcggctca gctctcggca ggcggcggcg ttgctcagcc 240
gagcgcagac gggaccctcg cagcgagacc tcagcgactc ctaaagtcaa aagttggcgg 300
cgggcgccgg gctccgcgcg ctctccacgg ccgctgcctc gcgtcgccgc cgcagccaag 360
gagggcagga gggagggggg tgggggcagc ggagggaggg gtgggaagca ccatgcagtt 420
tgtatcctgg gccacactgc taacgctcct ggtgcgggac ctggccgaga tggggagccc 480
agacgccgcg gcggccgtgc gcaaggacag gctgcacccg aggcaagtga aattattaga 540
gaccctgagc gaatacgaaa tcgtgtctcc catccgagtg aacgctctcg gagaaccctt 600
tcccacgaac gtccacttca aaagaacgcg acggagcatt aactctgcca ctgacccctg 660
gcctgccttc gcctcctcct cttcctcctc tacctcctcc caggcgcatt accgcctctc 720
tgccttcggc cagcagtttc tatttaatct caccgccaat gccggattta tcgctccact 780
gttcactgtc accctcctcg ggacgcccgg ggtgaatcag accaagtttt attccgaaga 840
ggaagcggaa ctcaagcact gtttctacaa aggctatgtc aataccaact ccgagcacac 900
ggccgtcatc agcctctgct caggaatgct gggcacattc cggtctcatg atggggatta 960
ttttattgaa ccactacagt ctatggatga acaagaagat gaagaggaac aaaacaaacc 1020
ccacatcatt tataggcgca gcgcccccca gagagagccc tcaacaggaa ggcatgcatg 1080
tgacacctca gaacacaaaa ataggcacag taaagacaag aagaaaacca gagcaagaaa 1140
atggggagaa aggattaacc tggctggtga cgtagcagca ttaaacagcg gcttagcaac 1200
agaggcattt tctgcttatg gtaataagac ggacaacaca agagaaaaga ggacccacag 1260
aaggacaaaa cgttttttat cctatccacg gtttgtagaa gtcttggtgg tggcagacaa 1320
cagaatggtt tcataccatg gagaaaacct tcaacactat attttaactt taatgtcaat 1380
tgtagcctct atctataaag acccaagtat tggaaattta attaatattg ttattgtgaa 1440
cttaattgtg attcataatg aacaggatgg gccttccata tcttttaatg ctcagacaac 1500
attaaaaaac ttttgccagt ggcagcattc gaagaacagt ccaggtggaa tccatcatga 1560
tactgctgtt ctcttaacaa gacaggatat ctgcagagct cacgacaaat gtgatacctt 1620
aggcctggct gaactgggaa ccatttgtga tccctataga agctgttcta ttagtgaaga 1680
tagtggattg agtacagctt ttacgatcgc ccatgagctg ggccatgtgt ttaacatgcc 1740
tcatgatgac aacaacaaat gtaaagaaga aggagttaag agtccccagc atgtcatggc 1800
tccaacactg aacttctaca ccaacccctg gatgtggtca aagtgtagtc gaaaatatat 1860
cactgagttt ttagacactg gttatggcga gtgtttgctt aacgaacctg aatccagacc 1920
ctaccctttg cctgtccaac tgccaggcat cctttacaac gtgaataaac aatgtgaatt 1980
gatttttgga ccaggttctc aggtgtgccc atatatgatg cagtgcagac ggctctggtg 2040
caataacgtc aatggagtac acaaaggctg ccggactcag cacacaccct gggccgatgg 2100
gacggagtgc gagcctggaa agcactgcaa gtatggattt tgtgttccca aagaaatgga 2160
tgtccccgtg acagatggat cctggggaag ttggagtccc tttggaacct gctccagaac 2220

atgtggaggg ggcatcaaaa cagccattcg agagtgcaac agaccagaac caaaaaatgg 2280
tggaaaatac tgtgtaggac gtagaatgaa atttaagtcc tgcaacacgg agccatgtct 2340
caagcagaag cgagacttcc gagatgaaca gtgtgctcac tttgacggga agcattttaa 2400
catcaacggt ctgcttccca atgtgcgctg ggtccctaaa tacagtggaa ttctgatgaa 2460
ggaccggtgc aagttgttct gcagagtggc agggaacaca gcctactatc agcttcgaga 2520
cagagtgata gatggaactc cttgtggcca ggacacaaat gatatctgtg tccagggcct 2580
ttgccggcaa gctggatgcg atcatgtttt aaactcaaaa gcccggagag ataaatgtgg 2640
ggtttgtggt ggcgataatt cttcatgcaa aacagtggca ggaacattta atacagtaca 2700
ttatggttac aatactgtgg tccgaattcc agctggtgct accaatattg atgtgcggca 2760
gcacagtttc tcaggggaaa cagacgatga caactactta gctttatcaa gcagtaaagg 2820
tgaattcttg ctaaatggaa actttgttgt cacaatggcc aaaagggaaa ttcgcattgg 2880
gaatgctgtg gtagagtaca gtgggtccga gactgccgta gaaagaatta actcaacaga 2940
tcgcattgag caagaacttt tgcttcaggt tttgtcggtg ggaaagttgt acaaccccga 3000
tgtacgctat tctttcaata ttccaattga agataaacct cagcagtttt actggaacag 3060
tcatgggcca tggcaagcat gcagtaaacc ctgccaaggg gaacggaaac gaaaacttgt 3120
ttgcaccagg gaatctgatc agcttactgt ttctgatcaa agatgcgatc ggctgcccca 3180
gcctggacac attactgaac cctgtggtac agactgtgac ctgaggtggc atgttgccag 3240
caggagtgaa tgtagtgccc agtgtggctt gggttaccgc acattggaca tctactgtgc 3300
caaatatagc aggctggatg ggaagactga gaaggttgat gatggttttt gcagcagcca 3360
tcccaaacca agcaaccgtg aaaaatgctc aggggaatgt aacacgggtg gctggcgcta 3420
ttctgcctgg actgaatgtt caaaaagctg tgacggtggg acccagagga gaagggctat 3480
ttgtgtcaat acccgaaatg atgtactgga tgacagcaaa tgcacacatc aagagaaagt 3540
taccattcag aggtgcagtg agttcccttg tccacagtgg aaatctggag actggtcaga 3600
gtgcttggtc acctgtggaa aagggcataa gcaccgccag gtctggtgtc agtttggtga 3660
agatcgatta aatgatagaa tgtgtgaccc tgagaccaag ccaacatcta tgcagacttg 3720
tcagcagccg gaatgtgcat cctggcaggc gggtccctgg ggacagtgca gtgtcacttg 3780
tggacaggga taccagctaa gagcagtgaa atgcatcatt gggacttata tgtcagtggt 3840
agatgacaat gactgtaatg cagcaactag accaactgat acccaggact gtgaattacc 3900
atcatgtcat cctcccccag ctgccccgga aacgaggaga agcacataca gtgcaccaag 3960
aacccagtgg cgatttgggt cttggacccc atgctcagcc acttgtggga aaggtacccg 4020
gatgagatac gtcagctgcc gagatgagaa tggctctgtg gctgacgaga gtgcctgtgc 4080
taccctgcct agaccagtgg caaaggaaga atgttctgtg acaccctgtg ggcaatggaa 4140
ggccttggac tggagctctt gctctgtgac ctgtgggcaa ggtagggcaa cccggcaagt 4200
gatgtgtgtc aactacagtg accacgtgat cgatcggagt gagtgtgacc aggattatat 4260
cccagaaact gaccaggact gttccatgtc accatgccct caaaggaccc cagacagtgg 4320
cttagctcag caccccttcc aaaatgagga ctatcgtccc cggagcgcca gccccagccg 4380
cacccatgtg ctcggtggaa accagtggag aactggcccc tggggagcat gttccagtac 4440
ctgtgctggc ggatcccagc ggcgtgttgt tgtatgtcag gatgaaaatg gatacaccgc 4500
aaacgactgt gtggagagaa taaaacctga tgagcaaaga gcctgtgaat ccggcccttg 4560
tcctcagtgg gcttatggca actggggaga gtgcactaag ctgtgtggtg gaggcataag 4620
aacaagactg gtggtctgtc agcggtccaa cggtgaacgg tttccagatt tgagctgtga 4680
aattcttgat aaacctcccg atcgtgagca gtgtaacaca catgcttgtc cacacgacgc 4740
tgcatggagt actggccctt ggagctcgtg ttctgtctct tgtggtcgag ggcataaaca 4800
acgaaatgtt tactgcatgg caaaagatgg aagccattta gaaagtgatt actgtaagca 4860
cctggctaag ccacatgggc acagaaagtg ccgaggagga agatgcccca aatggaaagc 4920
tggcgcttgg agtcagtgct ctgtgtcctg tggccgaggc gtacagcaga ggcatgtggg 4980
ctgtcagatc ggaacacaca aaatagccag agagaccgag tgcaacccat acaccagacc 5040
ggagtcggaa cgcgactgcc aaggcccacg gtgtcccctc tacacttgga gggcagagga 5100
atggcaagaa tgcaccaaga cctgcggcga aggctccagg taccgcaagg tggtgtgtgt 5160
ggatgacaac aaaaacgagg tgcatggggc acgctgtgac gtgagcaagc ggccggtgga 5220
ccgtgaaagc tgtagtttgc aaccctgcga gtatgtctgg atcacaggag aatggtcaga 5280
ggtaccgtcc tgggaactgt aaccatcgtc agctcagcca tggcctgaga gtggcagagg 5340
gatgagtgga gggatgagtg caggaatgtg ggagacttga ggctacccgc ccgatttgcc 5400
actgtgaact gtgtgttttc tgacaagtcc tcagctttcc caagctagaa ttccttgtat 5460
gcaaagcggg agagatgtaa gagatggtct ctaagtccct tcaggtctac attctgtgat 5520
tcaccttgat gtcctattgg cataaagaag aaattattac aggggctgca aactcatagg 5580
atgctgtgag gtgcctgaag acagttaagt ataagaaaat attgtagtgc caggatacaa 5640
acaaggagag atggcaactg tgacaaacta gcacatgctg tgtgaaggga gcagaatctc 5700
tttcactcca gctgtggcca tgcagaaatg tggtctagcg ttaccagacc tgatttttca 5760

agagaggcta aaaatctgga ctagtatgtg agatttccta acttgaaaat gggggctgaa 5820
atttttggtt ttaaaacatt gtaaggggca aacaaacccc tttcatgaac cagatgtgtt 5880
gtgcctgttt aacaaacagc ttcagaggaa gaaaataatt ttctataata tccgaagtat 5940
ctcaagtac 5949

<210> 2
<211> 1629
<212> PRT
<213> Human

<400> 2
Met Gln Phe Val Ser Trp Ala Thr Leu Leu Thr Leu Leu Val Arg Asp
1               5                   10                  15

Leu Ala Glu Met Gly Ser Pro Asp Ala Ala Ala Ala Val Arg Lys Asp
            20                  25                  30

Arg Leu His Pro Arg Gln Val Lys Leu Leu Glu Thr Leu Ser Glu Tyr
        35                  40                  45

Glu Ile Val Ser Pro Ile Arg Val Asn Ala Leu Gly Glu Pro Phe Pro
    50                  55                  60

Thr Asn Val His Phe Lys Arg Thr Arg Arg Ser Ile Asn Ser Ala Thr
65                  70                  75                  80

Asp Pro Trp Pro Ala Phe Ala Ser Ser Ser Ser Ser Ser Thr Ser Ser
                85                  90                  95

Gln Ala His Tyr Arg Leu Ser Ala Phe Gly Gln Gln Phe Leu Phe Asn
            100                 105                 110

Leu Thr Ala Asn Ala Gly Phe Ile Ala Pro Leu Phe Thr Val Thr Leu
        115                 120                 125

Leu Gly Thr Pro Gly Val Asn Gln Thr Lys Phe Tyr Ser Glu Glu Glu
    130                 135                 140

Ala Glu Leu Lys His Cys Phe Tyr Lys Gly Tyr Val Asn Thr Asn Ser
145                 150                 155                 160

Glu His Thr Ala Val Ile Ser Leu Cys Ser Gly Met Leu Gly Thr Phe
                165                 170                 175

Arg Ser His Asp Gly Asp Tyr Phe Ile Glu Pro Leu Gln Ser Met Asp
            180                 185                 190

Glu Gln Glu Asp Glu Glu Glu Gln Asn Lys Pro His Ile Ile Tyr Arg
        195                 200                 205

Arg Ser Ala Pro Gln Arg Glu Pro Ser Thr Gly Arg His Ala Cys Asp
    210                 215                 220

Thr Ser Glu His Lys Asn Arg His Ser Lys Asp Lys Lys Lys Thr Arg
225                 230                 235                 240

Ala Arg Lys Trp Gly Glu Arg Ile Asn Leu Ala Gly Asp Val Ala Ala
                245                 250                 255

Leu Asn Ser Gly Leu Ala Thr Glu Ala Phe Ser Ala Tyr Gly Asn Lys
            260                 265                 270

Thr Asp Asn Thr Arg Glu Lys Arg Thr His Arg Arg Thr Lys Arg Phe
        275                 280                 285

Leu Ser Tyr Pro Arg Phe Val Glu Val Leu Val Val Ala Asp Asn Arg
    290                 295                 300

Met Val Ser Tyr His Gly Glu Asn Leu Gln His Tyr Ile Leu Thr Leu
305                 310                 315                 320

Met Ser Ile Val Ala Ser Ile Tyr Lys Asp Pro Ser Ile Gly Asn Leu
                325                 330                 335

Ile Asn Ile Val Ile Val Asn Leu Ile Val Ile His Asn Glu Gln Asp
            340                 345                 350

Gly Pro Ser Ile Ser Phe Asn Ala Gln Thr Thr Leu Lys Asn Phe Cys
        355                 360                 365

Gln Trp Gln His Ser Lys Asn Ser Pro Gly Gly Ile His His Asp Thr
    370                 375                 380

Ala Val Leu Leu Thr Arg Gln Asp Ile Cys Arg Ala His Asp Lys Cys
385                 390                 395                 400

Asp Thr Leu Gly Leu Ala Glu Leu Gly Thr Ile Cys Asp Pro Tyr Arg
                405                 410                 415

Ser Cys Ser Ile Ser Glu Asp Ser Gly Leu Ser Thr Ala Phe Thr Ile
            420                 425                 430

Ala His Glu Leu Gly His Val Phe Asn Met Pro His Asp Asp Asn Asn
        435                 440                 445

Lys Cys Lys Glu Glu Gly Val Lys Ser Pro Gln His Val Met Ala Pro
    450                 455                 460

Thr Leu Asn Phe Tyr Thr Asn Pro Trp Met Trp Ser Lys Cys Ser Arg
465                 470                 475                 480

Lys Tyr Ile Thr Glu Phe Leu Asp Thr Gly Tyr Gly Glu Cys Leu Leu
                485                 490                 495

Asn Glu Pro Glu Ser Arg Pro Tyr Pro Leu Pro Val Gln Leu Pro Gly
            500                 505                 510

Ile Leu Tyr Asn Val Asn Lys Gln Cys Glu Leu Ile Phe Gly Pro Gly
        515                 520                 525

Ser Gln Val Cys Pro Tyr Met Met Gln Cys Arg Arg Leu Trp Cys Asn
    530                 535                 540

Asn Val Asn Gly Val His Lys Gly Cys Arg Thr Gln His Thr Pro Trp
545                 550                 555                 560

Ala Asp Gly Thr Glu Cys Glu Pro Gly Lys His Cys Lys Tyr Gly Phe

                565                 570                 575

Cys Val Pro Lys Glu Met Asp Val Pro Val Thr Asp Gly Ser Trp Gly
            580                 585                 590

Ser Trp Ser Pro Phe Gly Thr Cys Ser Arg Thr Cys Gly Gly Gly Ile
        595                 600                 605

Lys Thr Ala Ile Arg Glu Cys Asn Arg Pro Glu Pro Lys Asn Gly Gly
    610                 615                 620

Lys Tyr Cys Val Gly Arg Arg Met Lys Phe Lys Ser Cys Asn Thr Glu
625                 630                 635                 640

Pro Cys Leu Lys Gln Lys Arg Asp Phe Arg Asp Glu Gln Cys Ala His
                645                 650                 655

Phe Asp Gly Lys His Phe Asn Ile Asn Gly Leu Leu Pro Asn Val Arg
            660                 665                 670

Trp Val Pro Lys Tyr Ser Gly Ile Leu Met Lys Asp Arg Cys Lys Leu
        675                 680                 685

Phe Cys Arg Val Ala Gly Asn Thr Ala Tyr Tyr Gln Leu Arg Asp Arg
    690                 695                 700

Val Ile Asp Gly Thr Pro Cys Gly Gln Asp Thr Asn Asp Ile Cys Val
705                 710                 715                 720

Gln Gly Leu Cys Arg Gln Ala Gly Cys Asp His Val Leu Asn Ser Lys
                725                 730                 735

Ala Arg Arg Asp Lys Cys Gly Val Cys Gly Gly Asp Asn Ser Ser Cys
            740                 745                 750

Lys Thr Val Ala Gly Thr Phe Asn Thr Val His Tyr Gly Tyr Asn Thr
        755                 760                 765

Val Val Arg Ile Pro Ala Gly Ala Thr Asn Ile Asp Val Arg Gln His
    770                 775                 780

Ser Phe Ser Gly Glu Thr Asp Asp Asp Asn Tyr Leu Ala Leu Ser Ser
785                 790                 795                 800

Ser Lys Gly Glu Phe Leu Leu Asn Gly Asn Phe Val Val Thr Met Ala
                805                 810                 815

Lys Arg Glu Ile Arg Ile Gly Asn Ala Val Val Glu Tyr Ser Gly Ser
            820                 825                 830

Glu Thr Ala Val Glu Arg Ile Asn Ser Thr Asp Arg Ile Glu Gln Glu
        835                 840                 845

Leu Leu Leu Gln Val Leu Ser Val Gly Lys Leu Tyr Asn Pro Asp Val
    850                 855                 860

Arg Tyr Ser Phe Asn Ile Pro Ile Glu Asp Lys Pro Gln Gln Phe Tyr
865                 870                 875                 880

Trp Asn Ser His Gly Pro Trp Gln Ala Cys Ser Lys Pro Cys Gln Gly
                885                 890                 895

Glu Arg Lys Arg Lys Leu Val Cys Thr Arg Glu Ser Asp Gln Leu Thr
            900                 905                 910

Val Ser Asp Gln Arg Cys Asp Arg Leu Pro Gln Pro Gly His Ile Thr
        915                 920                 925

Glu Pro Cys Gly Thr Asp Cys Asp Leu Arg Trp His Val Ala Ser Arg
    930                 935                 940

Ser Glu Cys Ser Ala Gln Cys Gly Leu Gly Tyr Arg Thr Leu Asp Ile
945                 950                 955                 960

Tyr Cys Ala Lys Tyr Ser Arg Leu Asp Gly Lys Thr Glu Lys Val Asp
                965                 970                 975

Asp Gly Phe Cys Ser Ser His Pro Lys Pro Ser Asn Arg Glu Lys Cys
            980                 985                 990

Ser Gly Glu Cys Asn Thr Gly Gly Trp Arg Tyr Ser Ala Trp Thr Glu
        995                 1000                1005

Cys Ser Lys Ser Cys Asp Gly Gly Thr Gln Arg Arg Arg Ala Ile Cys
   1010                1015                1020

Val Asn Thr Arg Asn Asp Val Leu Asp Asp Ser Lys Cys Thr His Gln
1025                1030                1035                1040

Glu Lys Val Thr Ile Gln Arg Cys Ser Glu Phe Pro Cys Pro Gln Trp
                1045                1050                1055

Lys Ser Gly Asp Trp Ser Glu Cys Leu Val Thr Cys Gly Lys Gly His
            1060                1065                1070

Lys His Arg Gln Val Trp Cys Gln Phe Gly Glu Asp Arg Leu Asn Asp
        1075                1080                1085

Arg Met Cys Asp Pro Glu Thr Lys Pro Thr Ser Met Gln Thr Cys Gln
   1090                1095                1100

Gln Pro Glu Cys Ala Ser Trp Gln Ala Gly Pro Trp Gly Gln Cys Ser
1105                1110                1115                1120

Val Thr Cys Gly Gln Gly Tyr Gln Leu Arg Ala Val Lys Cys Ile Ile
                1125                1130                1135

Gly Thr Tyr Met Ser Val Val Asp Asp Asn Asp Cys Asn Ala Ala Thr
            1140                1145                1150

Arg Pro Thr Asp Thr Gln Asp Cys Glu Leu Pro Ser Cys His Pro Pro
        1155                1160                1165

Pro Ala Ala Pro Glu Thr Arg Arg Ser Thr Tyr Ser Ala Pro Arg Thr
   1170                1175                1180

Gln Trp Arg Phe Gly Ser Trp Thr Pro Cys Ser Ala Thr Cys Gly Lys
1185                1190                1195                1200

Gly Thr Arg Met Arg Tyr Val Ser Cys Arg Asp Glu Asn Gly Ser Val
                1205                1210                1215

Ala Asp Glu Ser Ala Cys Ala Thr Leu Pro Arg Pro Val Ala Lys Glu
            1220                1225                1230

Glu Cys Ser Val Thr Pro Cys Gly Gln Trp Lys Ala Leu Asp Trp Ser
        1235                1240                1245

Ser Cys Ser Val Thr Cys Gly Gln Gly Arg Ala Thr Arg Gln Val Met
    1250                1255                1260

Cys Val Asn Tyr Ser Asp His Val Ile Asp Arg Ser Glu Cys Asp Gln
1265                1270                1275                1280

Asp Tyr Ile Pro Glu Thr Asp Gln Asp Cys Ser Met Ser Pro Cys Pro
                1285                1290                1295

Gln Arg Thr Pro Asp Ser Gly Leu Ala Gln His Pro Phe Gln Asn Glu
            1300                1305                1310

Asp Tyr Arg Pro Arg Ser Ala Ser Pro Ser Arg Thr His Val Leu Gly
        1315                1320                1325

Gly Asn Gln Trp Arg Thr Gly Pro Trp Gly Ala Cys Ser Ser Thr Cys
    1330                1335                1340

Ala Gly Gly Ser Gln Arg Arg Val Val Val Cys Gln Asp Glu Asn Gly
1345                1350                1355                1360

Tyr Thr Ala Asn Asp Cys Val Glu Arg Ile Lys Pro Asp Glu Gln Arg
                1365                1370                1375

Ala Cys Glu Ser Gly Pro Cys Pro Gln Trp Ala Tyr Gly Asn Trp Gly
            1380                1385                1390

Glu Cys Thr Lys Leu Cys Gly Gly Gly Ile Arg Thr Arg Leu Val Val
        1395                1400                1405

Cys Gln Arg Ser Asn Gly Glu Arg Phe Pro Asp Leu Ser Cys Glu Ile
    1410                1415                1420

Leu Asp Lys Pro Pro Asp Arg Glu Gln Cys Asn Thr His Ala Cys Pro
1425                1430                1435                1440

His Asp Ala Ala Trp Ser Thr Gly Pro Trp Ser Ser Cys Ser Val Ser
                1445                1450                1455

Cys Gly Arg Gly His Lys Gln Arg Asn Val Tyr Cys Met Ala Lys Asp
            1460                1465                1470

Gly Ser His Leu Glu Ser Asp Tyr Cys Lys His Leu Ala Lys Pro His
        1475                1480                1485

Gly His Arg Lys Cys Arg Gly Gly Arg Cys Pro Lys Trp Lys Ala Gly
    1490                1495                1500

Ala Trp Ser Gln Cys Ser Val Ser Cys Gly Arg Gly Val Gln Gln Arg

1505                1510                1515                1520

His Val Gly Cys Gln Ile Gly Thr His Lys Ile Ala Arg Glu Thr Glu
                1525                1530                1535

Cys Asn Pro Tyr Thr Arg Pro Glu Ser Glu Arg Asp Cys Gln Gly Pro
            1540                1545                1550

Arg Cys Pro Leu Tyr Thr Trp Arg Ala Glu Glu Trp Gln Glu Cys Thr
        1555                1560                1565

Lys Thr Cys Gly Glu Gly Ser Arg Tyr Arg Lys Val Val Cys Val Asp
    1570                1575                1580

Asp Asn Lys Asn Glu Val His Gly Ala Arg Cys Asp Val Ser Lys Arg
1585                1590                1595                1600

Pro Val Asp Arg Glu Ser Cys Ser Leu Gln Pro Cys Glu Tyr Val Trp
                1605                1610                1615

Ile Thr Gly Glu Trp Ser Glu Val Pro Ser Trp Glu Leu
            1620                1625


<210> 3
<211> 25
<212> DNA
<213> Human

<400> 3
ggtggaatcc atcatgatac tgctg                                          25


<210> 4
<211> 25
<212> DNA
<213> Human

<400> 4
gaagctgata gtaggctgtg ttccc                                          25


<210> 5
<211> 25
<212> DNA
<213> Human

<400> 5
acagatggat cctggggaag ttgga                                          25


<210> 6
<211> 25
<212> DNA
<213> Human

<400> 6
ctgacataca acaacacgcc gctgg                                          25

<210> 7
<211> 21
<212> DNA
<213> Human

<400> 7
tcagagtgct tggtcacctg t                                                21


<210> 8
<211> 20
<212> DNA
<213> Human

<400> 8
cccgctttgc atacaaggaa                                                  20


<210> 9
<211> 19
<212> DNA
<213> Human

<400> 9
gagagcctca acaggaggc                                                   19


<210> 10
<211> 23
<212> DNA
<213> Human

<400> 10
gcactgagtg gaaaacccat tcc                                              23

## Description

### Field of Invention

The present invention relates to a member of the family of proteins known as ADAMTS.

### Background Of The Invention

ADAMTS proteins exhibit characteristics of the ADAM (A Disintegrin And Metalloprotease) family of metalloproteases, and in addition contain a thrombospondin domain (TS). The prototypic ADAMTS was identified in mouse, found to be expressed in heart and kidney and upregulated by proinflammatory stimuli (K. Kuno et al., *Molecular cloning of a gene encoding a new type of metalloproteinase-disintegrin family protein with thrombospondin motifs as a inflammation associated gene,* 272 Journal of Biological Chemistry 556 (January 1997)). To date nine members are recognized by the HUGO database (http://www.gene.ucl.ac.uk/users/hester/adamts.). Members of this family have the ability to degrade aggrecan, a high molecular weight proteoglycan which provides cartilage with important mechanical properties and which is lost during the development of arthritis.

Aggrecanase activity has been demonstrated for several ADAMTS proteins (See, e.g., M.D. Tortorella, *Purification and cloning of aggrecanase-1: a member of the ADAMTS family of proteins,* 284 Science 1664 (June 1999); I. Abbaszade, *Cloning and characterization of ADAMTS11*, *an aggrecanase from the ADAMTS family,* 274 Journal of Biological Chemistry 23443 (August 1999)). In addition to aggrecanase activity, ADAMTS-4 was shown to cleave another proteoglycan, brevican, found expressed predominately in the central nervous system (Matthews et al., *Brain-enriched hyaluronan binding (BEHAB)/Brevican cleavage in a glioma cell line is mediated by a disintegrin and metalloproteinase with thrombospondin motifs (ADAMTS) family member,* 275 Journal of Biological Chemistry 22695 (July 2000)). This activity was speculated to play a role in the invasiveness of glioma. Additional activities of ADAMTS-4 are proposed as its expression was induced in rat astrocytes treated with beta-amyloid, suggesting a role in Alzheimer's disease (Satoh et al., *ADAMTS-4 is transcriptionally induced in beta-amyloid treated rat astrocytes,* 289 Neuroscience Letters 177 (2000)). Other ADAMTS proteins are reported to exhibit antiangiogenic (See, e.g. F. Vazquez et al., *METH-1*, *A human ortholog of ADAMTS-1*, *and METH-2 are members of a new family of proteins with Angio-inhibitory activity,* 274 Journal of Biological Chemistry 23349 (August 1999)) and/or procollagen processing activities (A. Colige et al., *cDNA cloning and expression of bovine procollagen I N-proteinase: a new member of the superfamily of zinc-metalloproteinase with binding sites for cells and other matrix components,* 94 Proceedings of the National Academy of Sciences of the United States of America 2374 (March 1997)). Additional roles for ADAMTS-1 in fertility and organ development, particularly with respect to the urogenital system, were implicated by gene knockout experiments in mice (Shindo et al,, *ADAMTS-1: a metalloprotease-disintegrin essential for normal growth, fertility, and organ morphology and function,* 105 Journal of Clinical Investigation 1345 (May 2000)).

ADAMTS proteins and ADAMTS protein agonists and antagonists have important therapeutic uses, including treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetic shock, infertility and other diseases characterized by metalloproteinase activity and/or characterized by mammalian adamalysin activity.

WO 00/53774 describes an ADAMTS protein having a sequence related to that of the present invention.

### Summary Of The Invention

This invention relates to a novel ADAMTS protein, designated ADAMTS-SI, and to related polynucleotides and polypeptides. The invention also relates to production of the protein and polypeptides and to related assays. The invention further relates to methods for identifying substrates of the protein, for identifying inhibitors or activators of the protein, and to the use of the polypeptides or polynucleotides of the invention in diagnostic, biotherapeutic, and gene therapy methods.

In particular, the invention relates to an isolated polynucleotide molecule comprising a nucleotide sequence selected from the group consisting of:
(a) a nucleotide sequence having at least 80% identity to a nucleotide sequence encoding ADAMTS-SI polypeptide of SEQ ID NO: 2 or a metalloproteinase or disintegrin domain, prodomain, or thrombospondin (TSP) domain thereof;
(b) a nucleotide sequence of at least 15 contiguous nucleotides that hybridizes under stringent conditions to the polynucleotide molecule of SEQ ID NO: 1; and
(c) the complement of the nucleotide sequence of (a) or (b). Such an isolated polynucleotide molecule, can, for example, comprise DNA or RNA.

In one embodiment, the isolated polynucleotide is at least 80% identical to SEQ ID NO: 1, and in another embodiment comprises the ADAMTS-SI polypeptide encoding sequence of SEQ ID NO: 1, or a sequence encoding a metalloproteinase or disintegrin domain, prodomain, or thrombospondin (TSP) domain thereof.

In a further embodiment, the invention relates to a polypeptide encoded by the isolated polynucleotide molecule of the invention. For example, the ADAMTS-SI polypeptide of the invention can comprise an amino acid sequence which is at least 80% identical to SEQ ID NO: 2 or a metalloproteinase or disintegrin domain, prodomain, or thrombospondin (TSP) domain thereof, or an amino acid sequence of at least about 10 contiguous amino acids of ADAMTS-SI. In a preferred embodiment, the polypeptide comprises SEQ ID NO: 2 or a metalloproteinase or disintegrin domain, prodomain, or thrombospondin (TSP) domain thereof.

In another aspect, the invention relates to an expression system comprising a DNA or RNA molecule, wherein the expression system is capable of producing an ADAMTS-SI polypeptide that comprises an amino acid sequence that has at least 80% identity with a polypeptide of SEQ ID NO: 2 or a metalloproteinase or disintegrin domain, prodomain, or thrombospondin (TSP) domain thereof, when said expression system is present in a compatible host cell. In one embodiment of this aspect of the invention, the expression system is capable of producing an ADAMTS-SI polypeptide encoded by a polynucleotide of the invention.

In another aspect, the invention relates to a host cell that comprises the expression system of the invention.

In another aspect, the invention relates to a process for producing an ADAMTS-SI polypeptide that comprises culturing a host cell of the invention under conditions sufficient for production of the polypeptide, and recovering the polypeptide from cell culture.

In another aspect, the invention relates to a process for producing a cell which produces an ADAMTS-SI polypeptide comprising transforming or transfecting a host cell with an expression system of the invention such that the host cell, under appropriate culture conditions produces the ADAMTS-SI polypeptide.

In another aspect, the invention relates to an antibody that is immunospecific for an ADAMTS-SI polypeptide of the invention. The invention also relates to antagonists, agonists, and substrates of the polypeptide of the invention.

In a further aspect, the invention relates to a method for treating a subject in need of altering activity or expression of ADAMTS-SI comprising administering to the subject a therapeutically effective amount of an agonist or antagonist of ADAMTS-SI.

In another aspect, the invention relates to a method for treating a subject in need of altering activity or expression of ADAMTS-SI comprising administering to the subject a polynucleotide of the invention in order to alter said activity or expression. The invention also relates to a method for treating a subject in need of altering activity or expression of ADAMTS-SI comprising administering to the subject a therapeutically effective amount of a polypeptide that competes with ADAMTS-SI for its ligand, substrate, or receptor.

The invention also relates to a process for diagnosing a disease or a susceptibility to a disease in a subject related to expression or activity of ADAMTS-SI in a subject comprising determining presence or absence of a mutation in a nucleotide sequence encoding ADAMTS-SI in the genome of the subject. Alternately, the invention relates to a process for diagnosing a disease or a susceptibility to a disease in a subject related to expression or activity of ADAMTS-SI in a subject comprising analyzing for presence or amount of ADAMTS-SI expression in a sample derived from the subject.

In another aspect, the invention relates to a method for identifying compounds which antagonize ADAMTS-SI comprising:
(a) contacting a candidate compound with cells expressing an ADAMTS-SI polypeptide of the invention, or with cell membranes from cells expressing the ADAMTS-SI polypeptide, or the media conditioned by cells expressing the polypeptide, or a purified composition of said polypeptide; and
(b) determining inhibition of an ADAMTS-SI activity.

In an alternate embodiment, the invention relates to a method for identifying compounds which agonize ADAMTS-SI comprising:
(a) contacting a candidate compound with cells expressing an ADAMTS-SI polypeptide of the invention, or with cell membranes from cells expressing the polypeptide, or media conditioned by cells expressing the polypeptide, or a purified composition of the polypeptide; and
(b) determining stimulation of an ADAMTS SI activity.

In an further embodiment, the invention relates to a method for identifying compounds which bind to ADAMTS-SI comprising:
(a) contacting a candidate compound with cells expressing an ADAMTS-SI polypeptide of the invention, or with cell membranes from cells expressing the polypeptide, or the media conditioned by cells expressing the polypeptide, or a purified composition of the polypeptide; and
(b) determining binding of the candidate compound to the polypeptide.

The invention also relates to a method for detecting a polynucleotide encoding ADAMTS-SI in a biological sample containing nucleic acid material comprising:
(a) hybridizing an isolated polynucleotide of the invention that is specific to ADAMTS-SI to the nucleic acid material of the biological sample, thereby forming a hybridization complex; and
(b) detecting the hybridization complex, wherein presence of the hybridization complex correlates with the presence of the polynucleotide encoding ADAMTS-SI in the biological sample.

In a further embodiment, the invention relates to a method for identifying a substrate for ADAMTS-SI comprising contacting a polypeptide comprising an enzymatically active polypeptide of the invention with a candidate substrate and determining either conversion of substrate to product or binding of the polypeptide to the substrate.

The invention also relates to a method for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, or diabetic shock comprising administering a therapeutically effective amount of an agonist or antagonist, of ADAMTS-SI in combination with a pharmaceutically acceptable carrier.

The invention also relates to a method for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, or diabetic shock comprising administering a polypeptide of the invention in combination with a pharmaceutically acceptable carrier.

The invention also relates to a method for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising administering a polynucleotide of the invention in combination with a pharmaceutically acceptable carrier.

The invention further relates to a pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a therapeutically effective amount of an agonist or antagonist, of ADAMTS-SI in combination with a pharmaceutically acceptable carrier.

The invention also relates to a pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a polypeptide of the invention in combination with a pharmaceutically acceptable carrier.

The invention also relates to a pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a polynucleotide of the invention in combination with a pharmaceutically acceptable carrier.

### Brief Description Of The Drawings

FIG. 1 shows the complete polynucleotide sequence of ADAMTS-SI [SEQ ID NO:1].
FIG. 2 shows the substantially complete polypeptide sequence of ADAMTS-SI [SEQ ID NO:2].
FIG. 3 shows domains of the ADAMTS family of proteins, the sequences that correspond to those domains within the ADAMTS-SI polypeptide, and nucleic acid signature sequences.
FIG. 4 shows homology of the ADAMTS-SI polypeptide sequence of the metalloprotease domain aligned with those of other ADAMTS proteins.
FIG. 5 shows the results of a semi-quantitative PCR analysis of ADAMTS-SI expression and regulation by proinflammatory treatments in chondrocytes prepared from osteoarthritic cartilage.
FIG. 6 shows Western Blot analysis of purified recombinant ADAMTS-SI (amino acids 19-698) expressed in insect cells.

### Detailed Description Of The Invention

We have found relatively high levels of polynucleotide encoding the ADAMTS-SI protein in a cDNA library prepared from osteoarthritic cartilage, and lower levels in cDNA libraries derived from human lung and brain. The expression of ADAMTS-SI in osteoarthritic cartilage, and its modulation by proinflammatory agents, are consistent with its role in the pathology of arthritic disease.

### Definitions

The following definitions are provided to facilitate understanding of terms used herein.

"Antibodies" as used herein includes polyclonal and monoclonal antibodies, chimeric, single chain, and humanized antibodies, as well as Fab fragments, including the products of a Fab or other immunoglobulin expression library.

"Polynucleotide" generally refers to any polyribonucleotide or polydeoxribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is a mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example tritylated bases and unusual bases such as inosine. A variety of modifications have been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. "Polypeptides" may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquination, and they may be cyclic, with or without branching. Cyclic, branched and branched cyclic polypeptides may result from posttranslational natural processes or may be made by synthetic methods. Modifications include acetylation, acylation, ADP-ribosylation, amidation, covalent attachment of flavin, covalent attachment of a heme moiety, covalent attachment of phosphotidylinositol, crosslinking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, *Proteins - structure and molecular properties,* 2nd Ed., T.E. Creighton, W.H. Freeman and Company, New York, 1993; F. Wold, *Posttranslational protein modifications: perspectives and prospects,* pgs. 1-12 in Posttranslational covalent modification of proteins, B.C. Johnson, Ed., Academic Press, New York, 1983; S. Seifter and S. Englard, *Analysis for protein modifications and nonprotein cofactors,* 182 Methods of Enzymology 626 (1990); S.I. Rattan et al., *Protein synthesis, posttranslational modifications, and aging,* 663 Ann NY Acad Sci 48 (1992).

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions and/or deletions in any combination. A substituted or inserted amino acid residue may or may not be one encoded by the genetic code. A variant of a polynucleotide or a polypeptide may be naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" per se has an art-recognized meaning and can be calculated using published techniques. See, for example, *Computational molecular biology,* A.M. Lesk, ed., Oxford University Press, New York, 1988; *Biocomputing: informatics and genome projects,* D.W. Smith, ed., Academic Press, New York, 1993; *Computer analysis of sequence data, part* 1, A.M. Griffin, and H.G. Griffin, eds., Humana Press, New Jersey, 1994; *Sequence analysis in molecular biology,* G. von Heinje, ed., Academic Press, 1987; and *Sequence analysis primer,* M. Gribskov and J. Devereux, eds., M Stockton Press, New York, 1991. While there exist a number of methods to measure identity between two polynucleotide or polypeptide sequences, the term "identity" is well known to skilled artisans. Methods to determine identity and similarity are codified in computer programs. Preferred computer program methods to determine identity and similarity between two sequences include, but are not limited to, GCS program package; J. Devereux, et al., *A comprehensive set of sequence analysis programs for the VAX*, 12(1) Nucleic Acids Research 387 (January 1984); BLASTP; BLASTN; FASTA; S.F. Altschul et al., *Basic local alignment search tool*, 215(3) Journal of Molecular Biology 403 (October 1990). Among the methods stated above to determine identity, the preferred method is BLASTP.

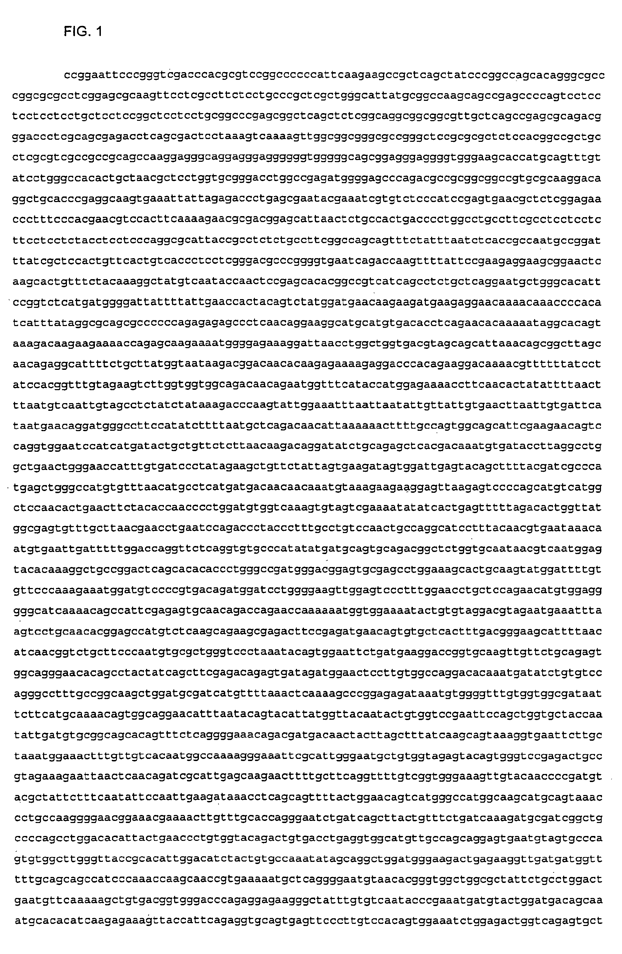

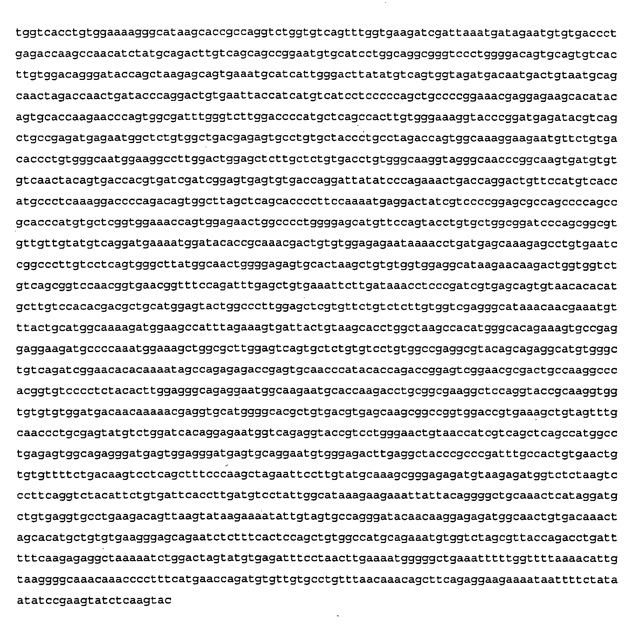

As an illustration, by a polynucleotide having a nucleotide sequence having at least, for example, 95% "identity" to a reference nucleotide sequence of FIG. 1, it is intended that the nucleotide sequence of the polynucleotide is identical to the reference sequence except that the polynucleotide sequence may include up to five point mutations per each 100 nucleotides of the reference nucleotide sequence of FIG. 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

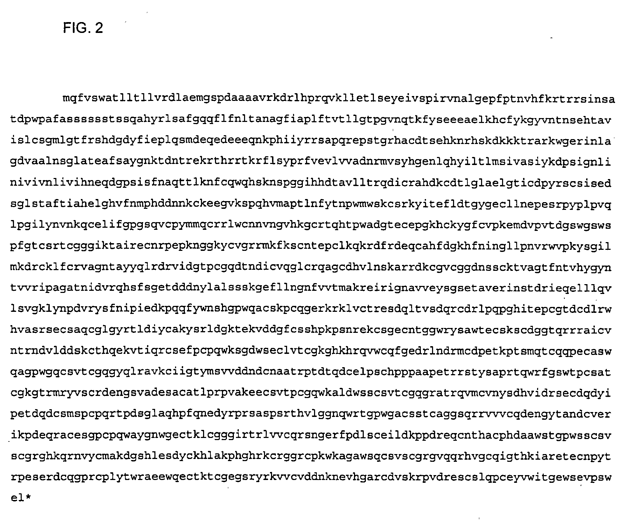

Similarly, by a polypeptide having an amino acid sequence having at least, for example, 95% "identity" to a reference amino acid sequence of FIG. 2, it is intended that the amino acid sequence of the polypeptide is identical to the reference sequence except that the polypeptide sequence may include up to five amino acid alterations per each 100 amino acids of the reference amino acid of FIG. 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### ADAMTS-SI Polypeptides

In one aspect, the present invention relates to ADAMTS-SI polypeptides. The ADAMTS-SI polypeptides include the polypeptide of FIG. 2, as well as polypeptides comprising an amino acid sequence of FIG. 2, and polypeptides comprising an amino acid sequence that has at least 80% identity to that of FIG. 2, preferably at least 90% identity, more preferably at least 95% identity to FIG. 2, and most preferably at least 97-99% identity to FIG. 2.

The ADAMTS-SI polypeptides may be in the form of an unprocessed or partially processed precursor, or the "mature" protein, which may in turn be a part of a larger protein such as a fusion protein. The mature form should normally begin with or near amino acid 104 and continue to the carboxyl terminus. It is often advantageous to include an additional amino acid sequence which contains secretory or leader sequences, pro-sequences, sequences which aid in purification or identification such as multiple histidine residues, or an additional sequence for stability during recombinant production.

Fragments of the ADAMTS-SI polypeptides are also included in the invention. A fragment is a polypeptide having an amino acid sequence that entirely is the same as part, but not all, of the amino acid sequence of the aforementioned ADAMTS-SI polypeptides. As with ADAMTS-SI polypeptides, fragments may be "free-standing," or comprised within a larger polypeptide of which they form a part or region, most preferably as a single continuous region.

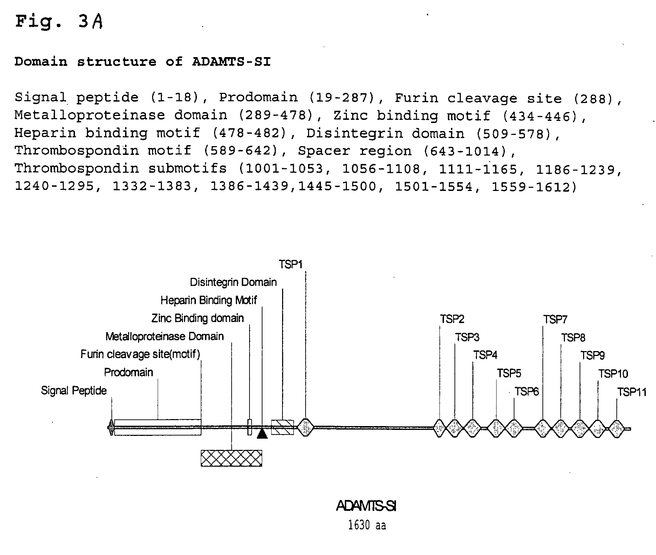

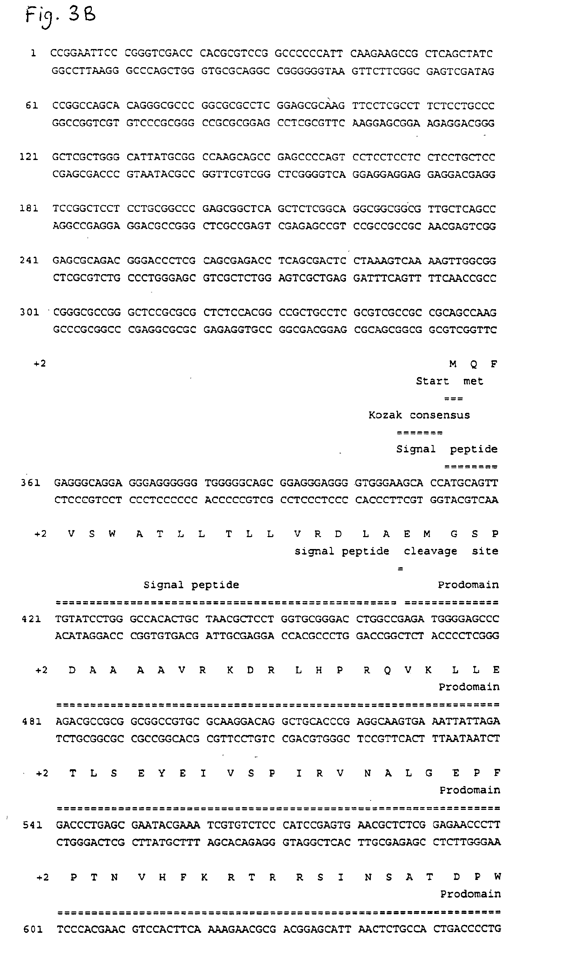

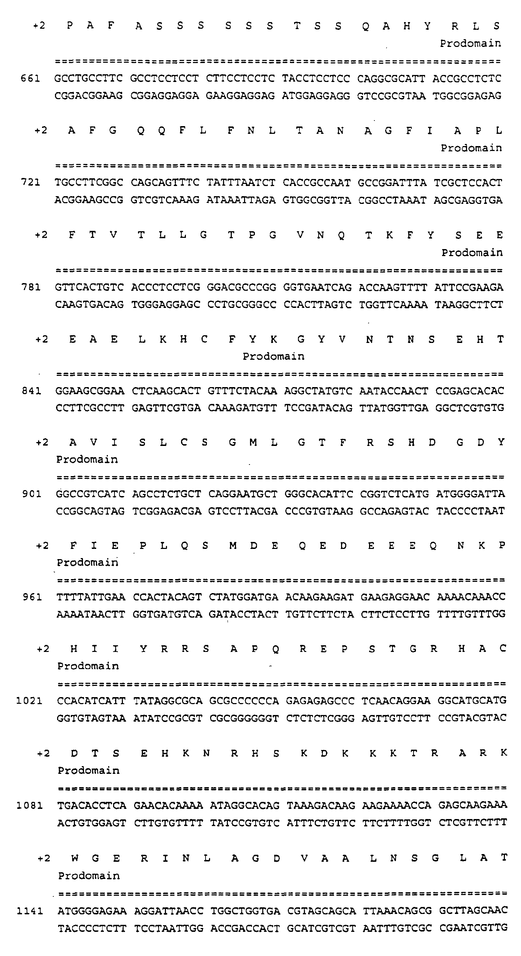

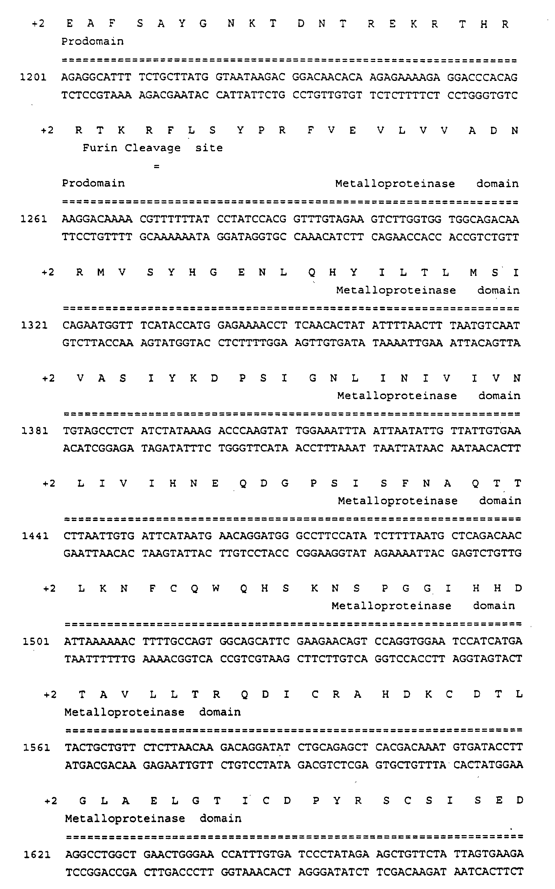

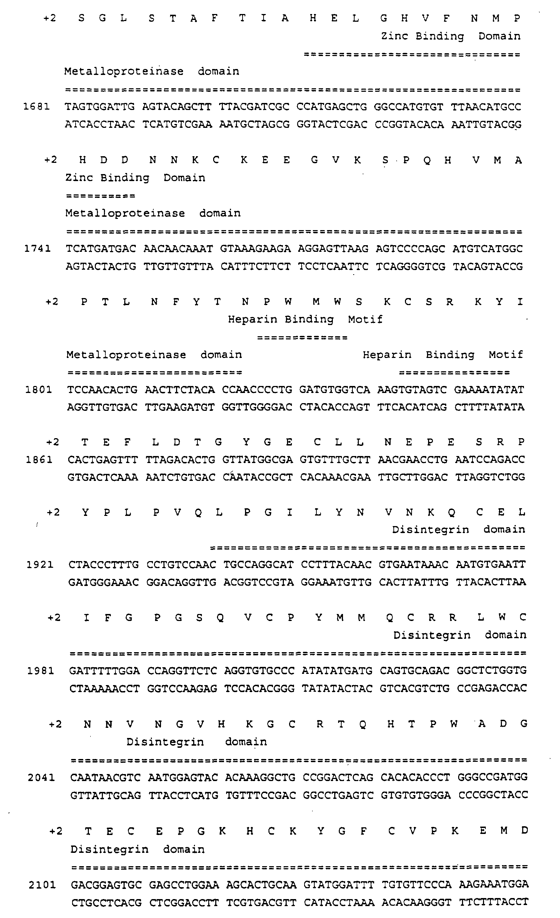

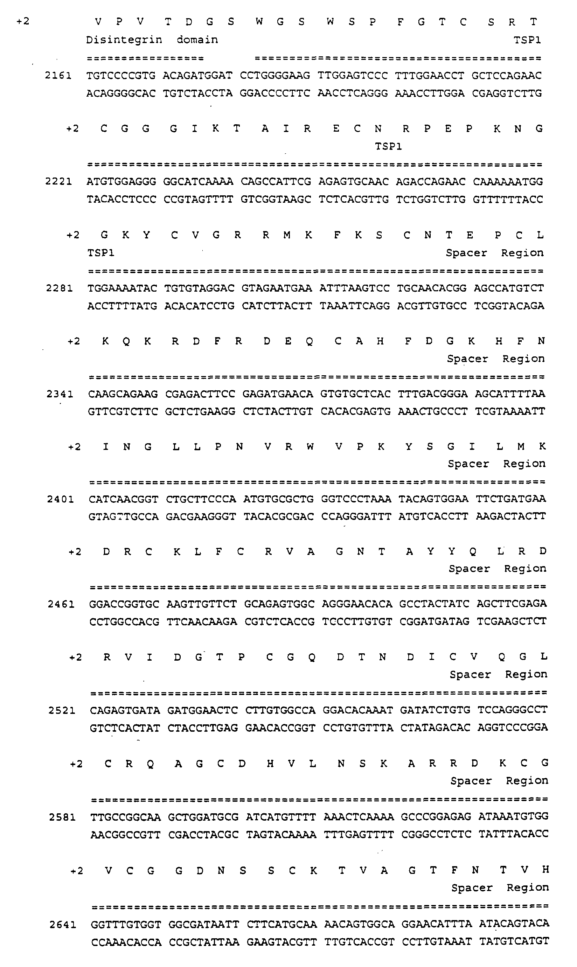

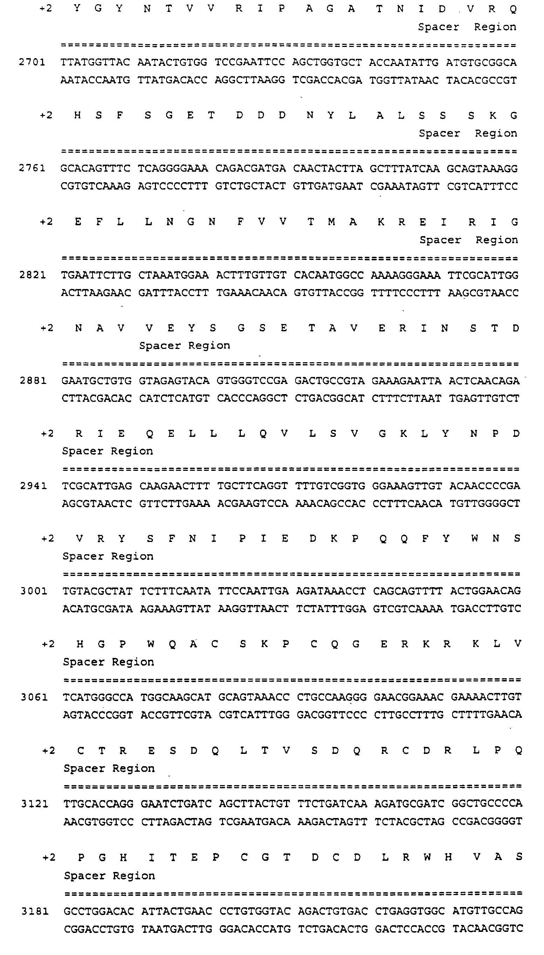

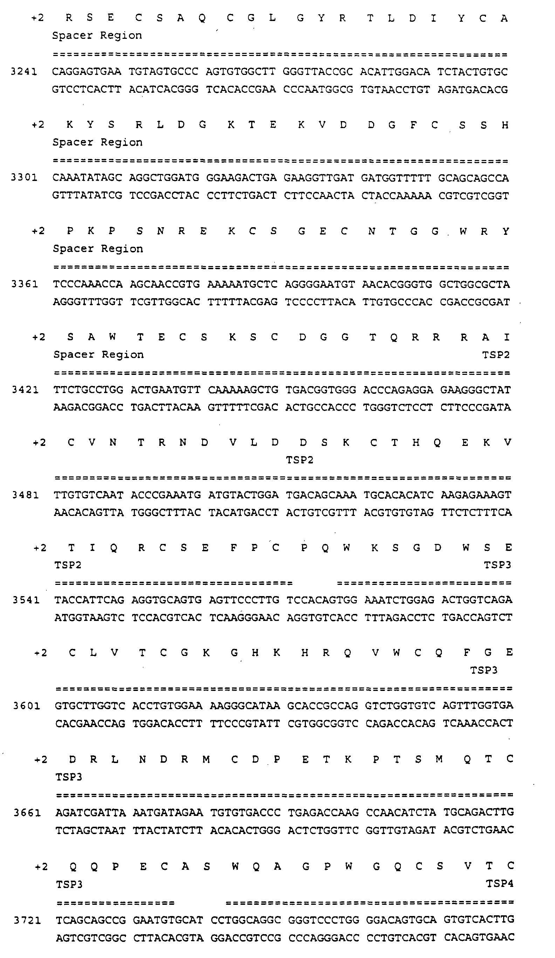

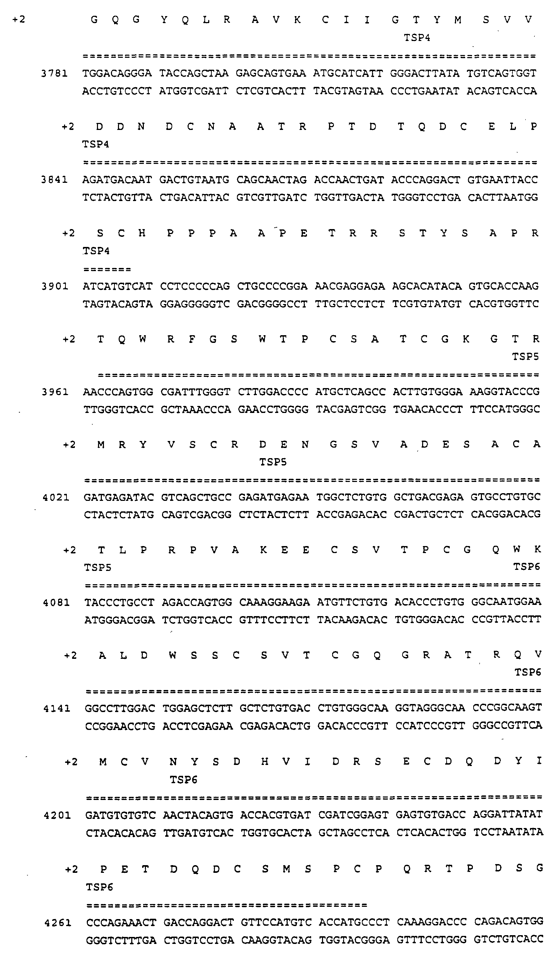

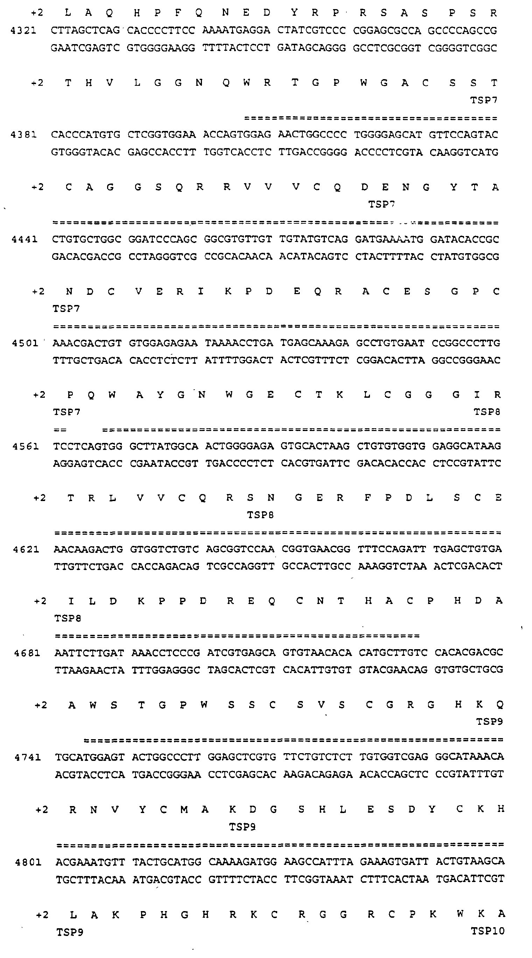

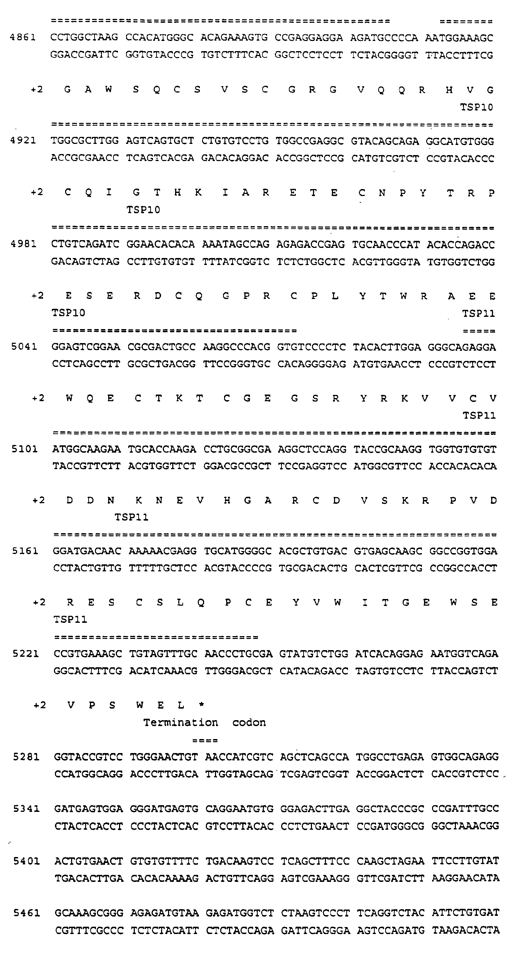

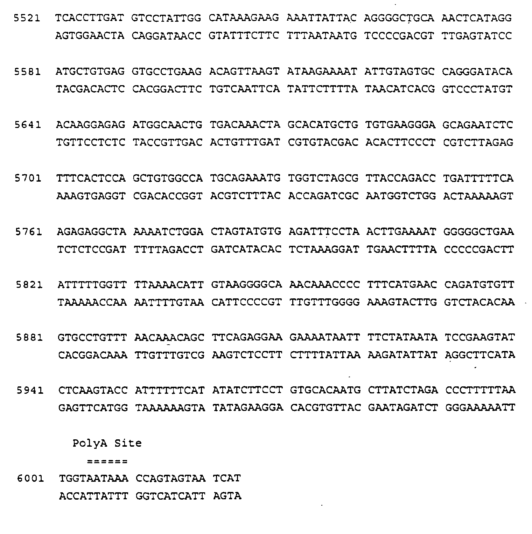

Preferred fragments include, for example, truncation polypeptides having the amino acid sequence of ADAMTS-SI polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Also preferred are biologically active fragments. Biologically active fragments are those that mediate one or more ADAMTS-SI activities, including those with a similar activity or an improved activity, or with a decreased undesirable activity. Most preferred are fragments that comprise one or more of the domains shown in FIG. 3. In particularly preferred embodiments, the fragment comprises the metalloproteinase domain, the disintegrin domain or the thrombospondin domain. In another embodiment, the polypeptide comprises amino acids 434 to 462 of SEQ ID NO: 2, which encompasses an extension of the zinc binding motif.

Such fragments are conventionally employed by themselves, or as part of fusion proteins. For example, expression vectors can be constructed that will express a fusion protein comprising a protein or polypeptide of the present invention. Such fusion proteins can be used, e.g., to raise antisera against the protein, to study the biochemical properties of the protein, to engineer a protein exhibiting different immunological or functional properties, to aid in the identification or purification, to improve the stability, of a recombinantly-expressed protein, or as therapeutic agents. Possible fusion protein expression vectors include but are not limited to vectors incorporating sequences that encode β-galactosidase and trpE fusions, maltose-binding protein fusions (pMal series; New England Biolabs), glutathione-S-transferase fusions (pGEX series; Pharmacia), polyhistidine fusions (pET series; Novagen Inc., Madison, WI), and thioredoxin fusions (pTrxFus; Invitrogen, Carlsbad, CA). As one example, the disintegrin domain or TSP domain, or a polypeptide comprising a variant or fragment thereof, may be administered alone, or as part of a fusion protein, to competitively inhibit in vivo or in vitro interactions with the native disintegrin domain or TSP domain. Methods are well-known in the art for constructing expression vectors encoding these and other fusion proteins.

Variants of the defined sequence and fragments also form part of the present invention. Preferred variants are those that vary from the referents by conservative amino acid substitutions, i.e., those that substitute a residue with another of like characteristics. Typical conservative substitutions are among Ala, Val, Leu and lle; among Ser and Thr; among the acidic residues Asp and Glu; among Asn and Gln; among the basic residues Lys and Arg; and among the aromatic residues Phe and Tyr. Particularly preferred are variants in which several, 5 to 10, 1 to 5, or 1 to 2 amino acids are substituted, deleted, or added in any combination.

The ADAMTS-SI polypeptides of the invention can be prepared in any suitable manner. The polypeptides include isolated naturally occurring polypeptides, recombinantly produced polypeptides, synthetically produced polypeptides, and polypeptides produced by a combination of these methods. These methods are well understood in the art.

Another embodiment of the present invention is an isolated ADAMTS-SI polypeptide. An isolated polypeptide is one that has been substantially removed from its natural milieu. As isolated ADAMTS-SI polypeptide can, for example, be obtained from its natural source, be produced using recombinant technology, or be synthesized chemically. An isolated ADAMTS-SI polypeptide can be full-length ADAMTS-SI polypeptide, the predicted mature form processed by furin cleavage of the prodomain (amino acid 292 with the predicted mature form beginning FLSY...), or any homologue of such a polypeptide, such as an ADAMTS-SI polypeptide in which amino acids have been deleted, inserted, inverted, substituted and/or derivatized (e.g., by glycosylation, phosphorylation, acetylation, myristoylation, prenylation, palmitoylation, amidation and/or addition of glycosylphosphatidyl inositol). A homologue of an ADAMTS-SI polypeptide is a polypeptide having an amino acid sequence that is sufficiently similar to a natural ADAMTS-SI polypeptide amino acid sequence that a nucleic acid sequence encoding the homologue is capable of hybridizing under stringent conditions to a nucleic acid sequence encoding the natural ADAMTS-SI polypeptide amino acid sequence disclosed herein. As used herein, "stringent hybridization conditions" refers to hybridization to filter-bound DNA in 5XSSC, 5X Denhardt's, 1% SDS, and 100ug/ml denatured salmon sperm DNA at 65°C, and washing in 0.1xSSC/0.1% SDS at 65°C (see Ausubel *et al.* (eds.), 1989, Current Protocols in Molecular Biology, Vol. I, Green Publishing Associates, Inc., and John Wiley & Sons, Inc., New York, at p. 2.10.3). A homologue of ADAMTS-SI polypeptide also includes a polypeptide having an amino acid sequence that is sufficiently cross-reactive such that the homologue has the ability to elicit an immune response against at least one epitope of naturally-occurring ADAMTS-SI polypeptide. Preferably the homologue retains one or more biological activities of ADAMTS-SI.

The minimal length of a protein homologue of the present invention is sufficient to be encoded by a nucleic acid molecule capable of forming a stable hybrid with the complementary sequence of a nucleic acid molecule encoding the corresponding natural protein. As such, the size of the nucleic acid molecule encoding such a protein homologue is dependent on nucleic acid composition, percent homology between the nucleic acid molecule and complementary sequence, as well as upon hybridization conditions per se (e.g., temperature, salt concentration and formamide concentration). The minimal size of such nucleic acid is typically at least about 12 to about 15 nucleotides in length if the nucleic acid molecules are GC-rich and at least about 15 to about 17 bases in length if they are AT-rich. The minimal size of a nucleic acid molecule used to encode an ADAMTS-SI protein homologue of the present invention is from about 20 to about 25 nucleotides in length. There is no limit, other than a practical limit, on the maximal size of such a nucleic acid molecule in that the nucleic acid molecule can include a portion of a gene, an entire gene, or multiple genes, or portions thereof. In one embodiment, the minimal size of an ADAMTS-SI protein homologue of the present invention is from 10, more preferably 12, even more preferably 25, amino acids in length. In another embodiment, a polypeptide of the invention comprises an amino acid sequence of more than about 10, or 25, preferably more than 75, more preferably more than 100, amino acids that is identical to an amino acid sequence of SEQ ID NO: 2. Preferred protein or polypeptide sizes depend on whether a full-length, multivalent protein (i.e., fusion protein having more than one domain each of which has a function), or a functional portion of such a protein is desired. Functional portions are obtainable based on the domains described herein, knowledge in the art concerning such domains and known assays for such domain, or its functional activity. Useful protein fragments or other polypeptides can also be screened for based on antigenic cross-reactivity with the ADAMTS-SI protein of SEQ ID NO: 2.

ADAMTS-SI protein homologues of the invention include allelic variations of the natural gene encoding the ADAMTS-SI protein. A "natural" gene is that found most often in nature. ADAMTS-SI protein homologues can be produced using techniques known in the art, including, but not limited to, direct modifications to a gene encoding a protein using, for example, classic or recombinant DNA techniques to effect random or targeted mutagenesis.

In another embodiment, an ADAMTS-SI polypeptide of the present invention comprises a portion of the ADAMTS-SI polypeptide disclosed herein, this portion having a molecular weight of about 25 kD (determined by Tris-glycine SDS-PAGE and resolved using methods standard in the art).

The present invention, encompasses the ADAMTS-SI proteins that have undergone posttranslational modification. Such modification can include, for example, glycosylation (e.g., including addition of N-linked and/or O-linked oligosaccharides) or posttranslational conformational changes or posttranslational deletions.

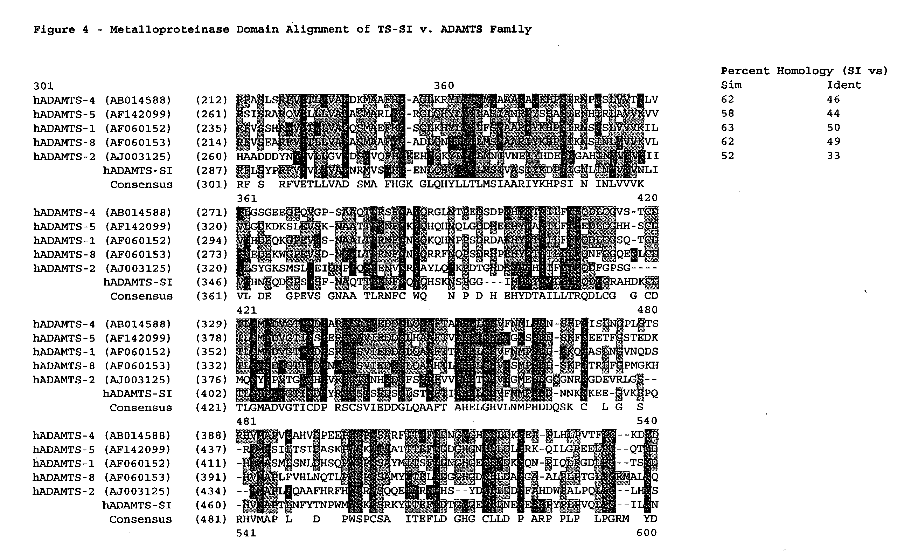

Based on the 29-36% identity in the metalloprotease domain of ADAMTS-SI as compared to other ADAMTS family members, ADAMTS-SI may have one or more proteolytic activities (e.g. collagenase, aggrecanase, procollagen protease) as well as anti-angiogenic activities that may or may not require the presence of the thrombospondin domains. See, FIGS. 3 and 4. These possible activities of ADAMTS-SI can be tested using techniques known to those skilled in the art. See, e.g., P.D. Brown et al., *Independent expression and cellular processing of Mr 72,000 type IV collagenase and interstitial collagenase in human* *tumorigenic cell lines,* 50(19) Cancer Research 6184 (October 1990); F. Vazquez et al., *METH-1 a human ortholog of ADAMTS-1, and METH-2 are members of a new family of proteins with angio-inhibitory activity,* 274 The Journal of Biological Chemistry 23349 (Aug. 1999); E.C. Arner et al., *Generation and characterization of aggrecanase,* 274 The Journal of Biological Chemistry 6594 (Mar. 1999); A. Colige et al., *cDNA cloning and expression of bovine procollagen I N-proteinase: A new member of the superfamily of zinc-metalloproteinases with binding sites for cell and other matrix components* 94 Proceedings of the National Academy of Sciences (USA) 2374 (March 1997).

### ADAMTS-SI Polynucleotides

Another aspect of the invention relates to ADAMTS-SI polynucleotides. ADAMTS-SI polynucleotides include isolated polynucleotides which encode the ADAMTS-SI polypeptides and fragments, and polynucleotides closely related thereto. More specifically, ADAMTS-SI polynucleotides of the invention include a polynucleotide comprising the nucleotide sequence set forth in FIG. 1 encoding a ADAMTS-SI polypeptide of FIG. 2, and a polynucleotide having the particular sequence of FIG. 1. ADAMTS-SI polynucleotides further include a polynucleotide comprising a nucleotide sequence that has at least 80% identity to a nucleotide sequence encoding the ADAMTS-SI polypeptide of FIG. 2, and a polynucleotide that is at least 80% identical to the polynucleotide sequence of FIG. 1. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred.

In one embodiment, the nucleic acid molecule of the invention has a nucleotide sequence has between 1 and 50, more preferably between 1 and 25, and most preferably between 1 and 5 nucleotides inserted, deleted, or substituted with respect to the sequence of SEQ ID NO: 1.

ADAMTS-SI polynucleotides of the invention also encompass nucleotide sequences which have sufficient identity to the nucleotide sequence contained in FIG. 1 to hybridize under conditions useable for amplification or for use as a probe or marker for ADAMTS-SI. Such sequences are typically 15 to 25 nucleotides in length with a target of 50% GC content and useful in PCR amplification or oligonucleotide hybridization methods well known to those skilled in the art. (See, e.g., Promega Protocols and Applications Guide, Third Edition, (1996), ISBN 1-8822474-57-1).

In one embodiment, the isolated nucleic acid molecule comprises a fragment of SEQ ID NO: 1 that is specific for ADAMTS-SI, i.e., specifically acts as a probe for SEQ ID NO: 1. The fragment may be at least, e.g., 15, 25, 35, 45 or 75 nucleotides in length.

Another embodiment of the present invention is an isolated nucleic acid molecule capable of hybridizing, under stringent conditions, with ADAMTS-SI polypeptide gene (FIG. 1) encoding an ADAMTS-SI protein of the present invention.

An isolated nucleic acid of the invention can include DNA, RNA or derivatives of either DNA or RNA.

An isolated nucleic acid molecule of the present invention can be obtained from its natural source either as an entire (i.e., complete) gene or a portion thereof capable of forming a stable hybrid with that gene. As used herein, the phrase "at least a portion of" an entity refers to an amount of the entity that is at least sufficient to have functional aspects of that entity. For example, at least a portion of a nucleic acid sequence, as used herein, is an amount of a nucleic acid sequence capable of forming a stable hybrid with a particular desired gene (e.g., ADAMTS genes) under stringent hybridization conditions. An isolated nucleic acid molecule of the present invention can also be produced using recombinant technology (e.g., polymerase chain reaction (PCR) amplification, cloning) or chemical synthesis. Isolated ADAMTS-SI protein nucleic acid molecules include natural nucleic acid molecules and homologues thereof, including, but not limited to natural allelic variants and modified nucleic acid molecules in which nucleotides have been inserted, deleted, substituted, and/or inverted in a manner that does not substantially interfere with the nucleic acid molecule's ability to encode an ADAMTS-SI protein of the present invention or to form stable hybrids under stringent conditions with natural nucleic acid molecule isolates encoding an ADAMTS-SI protein.

The invention also provides polynucleotides that are complementary to ADAMTS-SI polynucleotides described above.

### Expression of ADAMTS-SI

In one embodiment, an isolated ADAMTS-SI protein of the present invention is produced by culturing a recombinant cell capable of expressing the protein under conditions effective to produce the protein, and recovering the protein. Preferred cells include bacterial (e.g., *E. coli*), yeast (e.g., *Pichia),* insect (e.g., SF9) or mammalian, cells (e.g., CHO, Cos 7, and HEK 293). The recombinant cell is capable of expressing the ADAMTS-SI protein and is produced by transforming a host cell with one or more nucleic acid molecules of the present invention. Such recombinant cells are part of the present invention. Suitable transformation techniques include, but are not limited to, transfection, electroporation, microinjection, lipofection, adsorption and protoplast fusion. Recombinant cells of the invention may remain unicellular or may grow into a tissue organ or a multicellular organism. Nucleic acid molecules of the present invention used to transformed cells according to conventional techniques can remain extrachromosomal or can integrate into one or more sites within a chromosome of the transformed (i.e., recombinant) cell in such a manner that their ability to be expressed is retained.

Suitable host cells for transforming a cell include any cell capable of producing ADAMTS-SI proteins of the present invention after being transformed with at least one nucleic acid molecule of the present invention. Host cells can be either untransformed cells or cells that are already transformed with at least one nucleic acid molecule the present invention. Suitable host cells include bacterial, fungal (including yeast), insect, animal and plant cells.

The present invention also encompasses a recombinant vector which comprises a polynucleotide of the present invention inserted into a vector capable of delivering the polynucleotide into a host cell. Such a vector normally contains heterologous nucleic acid sequences, for example nucleic acid sequences that are not naturally found adjacent to ADAMTS-SI protein nucleic acid molecules of the present invention. The vector can be either DNA or RNA, and either prokaryotic or eukaryotic, and is typically a virus or a plasmid. Recombinant vectors can be used in cloning, sequencing, and/or otherwise manipulating or expressing ADAMTS-SI polynucleotides of the present invention.

In one embodiment of the invention, a recombinant cell is produced by transforming a host cell with one or more recombinant molecules, each comprising one or more polynucleotide molecules of the present invention operatively linked to an expression vector containing one or more transcription control sequences. The phrase "operatively linked" refers to a nucleic acid molecule inserted into an expression vector in a manner such that the molecule is able to be expressed when transformed into a host cell. As used herein, the phrase "expression vector" refers to a DNA or RNA vector that is capable of transforming a host cell and of effecting expression of a specified nucleic acid molecule.

Preferably, the expression vector is also capable of replicating within the host cell. Expression vectors can be either prokaryotic or eukaryotic, and are typically viruses or plasmids. Expression vectors of the present invention include vectors that effect direct gene expression in bacterial, fungal, insect, animal, and/or plant cells. Nucleic acid molecules of the present invention can be operatively linked to expression vectors containing regulatory sequences such as promoters, operators, repressors, enhancers, termination sequences, origins of replication, and other regulatory sequences that are compatible with the recombinant cell and that control the expression of nucleic acid molecules. Transcription control sequences that can be used in the present invention include those capable of controlling the initiation, elongation, and termination of transcription. Particularly important transcription control sequences are those which control transcriptional initiation, such as promoter, enhancer, operator and repressor sequences. Suitable transcription control sequences include those that function in one of the recombinant cells of the present invention. A variety of such transcription control sequences are known to those skilled in the art. Preferred transcription control sequences include those which function in bacterial yeast and mammalian cells, such as, but not limited to, tac, lac, trp, trc, oxy-pro, omp/lpp, rmB, bacteriophage lambda (λ) (such as λ_{P} and λP_{R} and fusions that include such promoters), bacteriophage T7, T7lac, bacteriophage T3, bacteriophage SP6, bacteriophage SPO1, metallothionein, alpha mating factor, baculovirus, vaccinia virus, herpesvirus, poxvirus, adenovirus, simian virus 40, retrovirus action, retroviral long terminal repeat, Rous sarcoma virus, heat shock, phosphate and nitrate transcription control sequences, as well as other sequences capable of controlling gene expression in prokaryotic or eukaryotic cells. Additional suitable transcription control sequences include tissue-specific promoters and enhancers as well as lymphokine-inducible promoters (e.g., promoters inducible by interferons or interleukins). Transcription control sequences useful in practicing the present invention include naturally occurring sequences associated with DNA encoding an ADAMTS-SI protein.

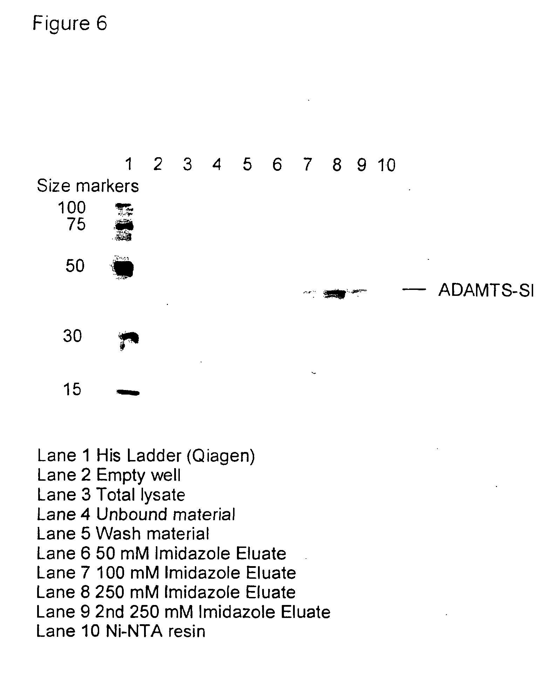

Preferred nucleic acid molecules for insertion into an expression vector include nucleic acid molecules encoding at least a portion of an ADAMTS-SI protein, or a homologue thereof. Expression vectors of the present invention may also contain fusion sequences, e.g., as discussed above, which allow expression of nucleic acid molecules of the present invention as fusion proteins. Inclusion of a fusion sequence in an ADAMTS-SI nucleic acid molecule of the present invention can enhance stability during production, storage, or use of the protein encoded by the nucleic acid molecule. Furthermore, a fusion segment can simplify purification of an ADAMTS-SI protein, enabling purification by affinity chromatography (for example see FIG. 6). Fusion segments can be of any size that affords the desired function (e.g., increased stability and/or easier purification). It is within the scope of the present invention to use one or more fusion segments. Fusion segments can be joined to amino and/or carboxyl termini of the ADAMTS-SI protein or polypeptide of the present invention. Linkages between fusion segments and ADAMTS-SI proteins can be constructed to be susceptible to cleavage to enable straightforward recovery of the ADAMTS-SI proteins. Fusion proteins are preferably produced by culturing a recombinant cell transformed with nucleic acid sequences that encode the fusion segment attached to either the carboxyl and/or amino terminal end of a ADAMTS-SI polypeptide of the invention.

The present invention includes recombinant cells resulting from transformation with a nucleic acid molecule of the present invention. Preferred recombinant cells are transformed with a nucleic acid molecule that encodes at least a portion of an ADAMTS-SI protein, or a homologue thereof. Amplifying the copy number of nucleic acid sequences of the invention can be accomplished by increasing the copy number of the nucleic acid sequence in the cell's genome or by introducing additional copies of the nucleic acid sequence by transformation. Copy number amplification is conducted in a manner such that greater amounts of enzyme are produced, leading to enhanced conversion of substrate to product. Transformation can be accomplished using any process by which nucleic acids are transformed into cells to enhance enzyme synthesis. Prior to transformation, the nucleic acid sequence can, if desired, be manipulated to encode an enzyme having a higher specific activity.

In accordance with the present invention, recombinant cells are used to produce an ADAMTS-SI protein of the present invention by culturing such cells under conditions effective to produce such a protein, and the protein recovered. Effective conditions include, but are not limited to, appropriate media, bioreactor, temperature, pH and oxygen conditions that permit protein production. Suitable media are typically aqueous and comprise assimilable carbohydrate, nitrogen and phosphate sources, as well as appropriate salts, minerals, metals and other nutrients, such as vitamins. The medium may comprise complex nutrients, or may be minimal.

Cells of the present invention can be cultured in conventional fermentation bioreactors, which include, but are not limited to, batch, fed-batch, cell recycle, and continuous fermentors. Culturing can also be conducted in shake flasks, test tubes, microtiter dishes, and petri plates. Culturing is carried out at a temperature, pH and oxygen content appropriate for the recombinant cell. Such culturing is within the expertise of one of ordinary skill in this art.

Depending on the vector and host system used for production, resultant ADAMTS-SI proteins may either remain within the recombinant cell or be secreted into the fermentation medium. "Recovering the protein" according to the invention may involve simply collecting the fermentation medium or cells containing the protein and need not include additional steps of separation or purification. ADAMTS-SI proteins of the present invention can be purified using a variety of standard protein purification techniques, such as, but not limited to, affinity chromatography (FIG. 6), ion exchange chromatography, filtration, electrophoresis, hydrophobic interaction chromatography, gel filtration chromatography, reverse phase chromatography, chromatofocusing and differential solubilization.

In addition, an ADAMTS-SI protein of the present invention can be produced by isolating the ADAMTS-SI protein from cells expressing the ADAMTS-SI protein recovered from transgenic animal, or from fluid, such as milk, recovered from such an animal. An isolated protein or polypeptide of the present invention can be used to formulate a therapeutic composition as discussed further below.

### Antibodies to ADAMTS-SI

The present invention also includes antibodies capable of selectively binding to an ADAMTS-SI protein or polypeptide of the present invention. Polyclonal populations of anti-ADAMTS-SI antibodies can be contained in an ADAMTS-SI antiserum. Binding can be measured using a variety of methods known to those skilled in the art including immunoblot assays, immunoprecipitation assays, enzyme immunoassays (e.g., ELISA), radioimmunoassay, immonofluorescent antibody assays and immunoelectron microscopy; see, for example, Sambrook et al., *Molecular cloning: a laboratory manual,* Cold Spring Harbor Labs Press, 1989.

Antibodies of the present invention can be either monoclonal or polyclonal antibodies and can be prepared using techniques standard in the art. Antibodies of the present invention include functional equivalents such as antibody fragments and genetically-engineered antibodies, including single chain antibodies that are capable of selectively binding to at least one of the epitopes of the protein used to obtain antibodies. Preferably, antibodies are raised in response to proteins that are encoded, at least in part, by an ADAMTS-SI nucleic acid molecule.

### Identification of ADAMTS-SI substrates

The present invention also encompasses methods for identifying ADAMTS-SI substrates. Such methods include those wherein a candidate substrate is contacted with a polypeptide comprising an enzymatically active ADAMTS-SI polypeptide of the invention, and conversion of substrate to product is determined, or binding of polypeptide to the candidate substrate determined. The invention also encompasses rational drug design conducted using computer software that calculates interactions between candidate compounds and polypeptides or polynucleotides of the invention.

Substrates may be identified by a candidate protein or synthetic substrate approach. For example, candidate proteins can be cast within an agarose gel matrix and the ability of the ADAMTS-SI protein to digest the protein determined using protein zymography. See, P.D. Brown et al., *Independent expression and cellular processing of Mr 72,000 type IV collagenase and interstitial collagenase in human tumorigenic cell lines,* 50(19) Cancer Research 6184 (October 1990). Alternatively, a phage display or fluorometric peptide library can be screened to identify substrates of the protein. See, D.R. O'Boyle et al., *Identification of a novel peptide substrate of HSV-1 protease using substrate phage display,* 236(2) Virology 338 (September 1997).

### Agonists/antagonists of ADAMTS-SI

The present invention also includes assays for determining agonists and/or antagonists of ADAMTS-SI. Assays for determining aggrecanase, collagenase, procollagen protease and/or angiogenic activities may be used to identify agonist or antagonist compounds, preferably small molecular weight compounds of less than 700 daltons. The compounds may contain a hydroxamic acid moiety or an optionally substituted heterocyclic nucleus, or an aryl or heteroaryl sulfonamide moiety, which compounds inhibit or stimulate the activity of endogenous or recombinant ADAMTS-SI. E.C. Arner et al., *Generation and characterization of aggrecanase. A soluble, cartilage-derived aggrecan-degrading activity,* 274 Journal of Biological Chemistry 6594 (March 1999); M.D. Tortorella et al., *supra;* A. Colige et al., *supra*;Vazquez et al.,, *supra.* ELISA or fluorescent substrate assays can be performed to determine agonists or antagonists, or to determine specific proteolytic activity, of an ADAMTS-SI protein.

### Diagnostic Assays

The present invention also includes processes for diagnosing diseases or susceptibility to diseases related to expression and/or activity of ADAMTS-SI. Such diseases may be identified by determining the presence or absence of a mutation in the nucleotide sequence encoding said ADAMTS-SI polypeptide in the genome of a patient. Alternately, the presence or amount of ADAMTS-SI polypeptide in a sample derived from a patient may be determined as an indicator of disease or susceptibility to disease. Such diagnosis may be performed for diseases including the following: arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetic shock, infertility and other diseases characterized by metalloproteinase activity and/or characterized by mammalian adamalysin activity.

### Therapeutic compositions and uses of ADAMTS-SI

In one embodiment of the present invention, an antibody, agonist, antagonist, substrate and/or variant of ADAMTS-SI, or a polypeptide or polynucleotide of the invention, is employed in a therapeutic composition for treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetic shock, infertility or other diseases characterized by metalloproteinase activity and/or mammalian adamatysin activity.

In one embodiment, polynucleotides of the invention can, for example, be employed to transform cells in gene therapy application, e.g., as part of in vivo or ex vivo gene therapy. Polynucleotides can also be employed in antisense therapy, and in the construction of ribozymes. Use of polynucleotides in these methods is known to those skilled in this art.

For administration to mammals, including humans, a variety of conventional routes may be used including oral, parenteral (e.g., intravenous, intramuscular or subcutaneous), buccal, anal and topical.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelation and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof. In the case of animals, they are advantageously contained in an animal feed or drinking water in a concentration of 5-5000 ppm, preferably 25 to 500 ppm.

For parenteral administration (intramuscular, intraperitoneal, subcutaneous and intravenous use) a sterile injectable solution of the active ingredient is usually prepared. Solutions of the therapeutic compound in either sesame or peanut oil or in aqueous propylene glycol may be employed. The aqueous solutions should be suitably adjusted and buffered, preferably at a pH of greater than 8, if necessary and the liquid diluent first rendered isotonic. These aqueous solutions are suitable intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art. In the case of animals, compounds can be administered intramuscularly or subcutaneously at dosage levels of about 0.1 to 50 mg/kg/day, advantageously 0.2 to 10 mg/kg/day given in a single dose or up to 3 divided doses.

Active compounds may also be formulated in rectal compositions such as suppositories or retention enemas, e.g., containing conventional suppository bases such as cocoa butter or other glycerides.

For intranasal administration or administration by inhalation, active compounds are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A therapeutic composition of the present invention can be administered to any subject having a medical disorder as herein described. Acceptable protocols by which to administer therapeutic compounds of the present invention in an effective manner vary according to individual dose size, number of doses, frequency of dose administration and mode of administration. Determination of appropriate protocols is accomplished by those skilled in the art without undue experimentation. An effective dose refers to a dose capable of treating a subject for a medical disorder as described herein. Effective doses vary depending upon, for example, the therapeutic composition used, the medical disorder being treated and the size and type of recipient animal.

The dosage and length of treatment depends on the disease state being treated. The duration of treatment may be a day, a week or longer and may last over the lifetime of the patient.

### Identification of ADAMTS-SI

For identification of novel ADAMTS gene family members, a non-redundant set of publicly available protein sequences was assembled (accession numbers: D67076, AJ003125, AB002364, AB014588). A series of low stringency BLAST searches were then performed against the LifeSeq Gold™ database (Incyte) using each of the above protein sequences as queries and RecA-mediated homology capture used to isolate corresponding cDNA clones. Colonies hybridizing to the capture sequence were isolated and the largest clone (S3-2.6 kb) was sequenced. The sequence encoded a signal peptide, prodomain, furin cleavage site, and partial metalloproteinase domain. However, the Zinc binding motif, disintegrin, and thrombospondin motifs, were not present. Subsequently, attempts to clone ADAMTS family members led to the identification of a clone (I1A). Sequencing of this clone revealed 55 amino acids of overlapping sequence identity to ADAMTS-S and encoded the remaining metalloproteinase domain. Thus, by pursuing the cloning of two independent ESTs, the sequences collapsed into a single novel ADAMTS termed ADAMTS-SI (nucleotides 1-1385 of FIG.1 [SEQ ID NO: 1]). A published unannotated sequence (GenBank accession #AB037733) was found to overlap with our composite ADAMTS-SI clone and completed the identification of a full-length sequence for ADAMTS-SI [SEQ ID NO: 1]). Utilizing RT-PCR, the full-length sequence for ADAMTS-SI was cloned and sequenced in a series of overlapping fragments. The complete polypeptide sequence of ADAMTS-SI is shown in FIG. 2 [SEQ ID NO: 2]. Fragments comprising the nucleotides 1544-4480 of SEQ ID NO: 1 were amplified with the following primer sets from human osteoarthritic cartilage RNA: Nucleotides 1544-2516 of SEQ ID NO: 1 (sense 5'GGTGGAATCCATCATGATACTGCTG3' [SEQ ID NO: 3];antisense 5'GAAGCTGATAGTAGGCTGTGTTCCC3' [SEQ ID NO: 4]) Nucleotides 2171-4480 of SEQ ID NO: 1 (sense 5'ACAGATGGATCCTGGGGAAGTTGGA3' [SEQ ID NO: 5]; antisense 5'CTGACATACAACAACACGCCGCTGG3' [SEQ ID NO: 6]) Also, the 3' terminus comprising nucleotides 3596-5470 of SEQ ID NO: 1 was cloned by RT-PCR from A549 (human lung cell line) RNA with the following primer set: (sense 5'TCAGAGTGCTTGGTCACCTGT3' [SEQ ID NO: 7]; antisense 5'CCCGCTTTGCATACAAGGAA3' [SEQ ID NO. 8]). The RT-PCR performed to generate these overlapping fragments utilized the One-Step System (GIBCO, Gaithersburg,) according to the manufacturer's instructions with the addition of Elongase polymerase (Roche, Indianapolis, IN). Briefly, 1 ug of total RNA was used in each reaction containing 200 nM of sense and antisense gene-specific primers with 1 ul of Elongase polymerase added. The thermal cycling parameters were: 1 cycle at 50°C for 30 minutes, 94°C for 2 minutes, followed by 35 cycles of: 94°C 15 sec, 55°C 30 sec, and 72°C 6 minutes.

### Expression of ADAMTS-SI in Chondrocytes Derived from Osteoarthritic (OA) Cartilage and Induction by Proinflammtory Cytokines

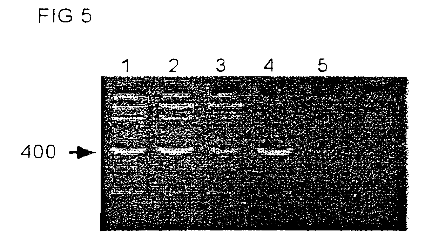

Primers to ADAMTS-SI were synthesized by LifeTechnologies (sense primer 5'-GAGAGCCTCAACAGGAGGC-3' [SEQ ID NO: 9 and antisense primer 5'-GCACTGAGTGGAAAACCCATTCC-3') [SEQ ID NO: 10]. Chondrocytes were isolated from the cartilage of two patients with osteoarthritis and grown in tissue culture media. Chondrocytes were mock treated (lane 1 = patient 1, lane 3 = patient 2 in Figure 5) or treated with retinoic acid (lane 2, patient 1 in Figure 5) or IL-1 (lane 4, patient 2 in Figure 5). RNA was isolated by standard methods and a 5 ug of each sample was used in a first strand cDNA synthesis reaction using oligo dT priming (using Superscript reverse transcriptase and conditions recommended by the manufacturer). 4% of the cDNA reaction products were used as template in a PCR reaction using PCR supermix (LTI) and 150 ng of the above primers using conditions recommended by the manufacturer. PCR cycling conditions 95°C 60 seconds followed by 30 cycles of: 94° C 30 seconds, 54° C 30 seconds, 72° C 45 seconds. Reaction products (10ul) were fractionated by agarose gel electrophoresis (4% NuSieve, FMC) and the ethidium bromide stained gel visualized by UV. PCR controls (water, lane 5 in Figure 5) and DNA molecular weight markers (not shown, PhiX phage DNA digested with HaeIII, LTI) were included. A product, indicated by the arrow and corresponding to the predicted size (~400 nucleotides) was observed in both patients and upregulated by treatment with proinflammatory agents.

### Expression and Purification of a Truncated ADAMTS-SI Fusion Protein

A truncated version of ADAMTS-SI which included the pro, metalloprotease, disintegrin and first thrombospondin domains (amino acids 19-698 of SEQ. ID NO: 2) was cloned as a fusion protein with a heterologous amino-terminal secretory signal (from the human chitinase-like protein) and a carboxyl-terminal 6 histidine tag into a baculovirus expression vector (pFastBac derivative, GIBCO BRL, Gaithersburg, MD), and purified by nickel affinity chromatography and detected by Western blot using an antibody raised against the histidine tag epitope (Qiagen Inc., Valencia, CA). FIG. 6. The size of the expressed product (45 kD) was consistent with proper maturation of the protein by furin cleavage of the prodomain.

In particular, approximately 100 ml of conditioned insect cell media was dialyzed against PBS containing 20 mM imidazole and purified by batch affinity chromatography overnight at 4°C. The resin was washed with phosphate buffer containing 20 mM imidazole and eluted step-wise with phosphate buffer containing 50 mM, 100 mM, and finally 250 mM imidazole. Samples were diluted 2x with sample buffer (Novex, Carlsbad, CA) containing 5 % B-mercaptoethanol denatured by boiling for 2 minutes. 20 ul of each sample was resolved on 4-20% gradient SDS-PAGE mini-gel and transferred to Nitrocellulose (Novex, Carlsbad).

BLASTP 2.0.9 analysis showed high homology to a number of ADAMTS family members, as indicated in the alignment in Figure 4.

The foregoing description of the invention has been presented for purposes of illustration and description. Further, the description is not intended to limit the invention to the form disclosed herein. Consequently, variations and modifications commensurate with the above teachings, and the skill or knowledge in the relevant art are within the scope of the present invention. It is intended that the appended claims be construed to include alternate embodiments to the extent permitted by the prior art.

### Sequence ID Listing

SEQ ID NO: 1 is the nucleotide sequence of ADAMTS-SI.

SEQ ID NO: 2 is the deduced amino acid sequence of ADAMTS-SI.

SEQ ID NOS: 3-10 are nucleotide sequences of primers described in the Examples.

## Claims

1. An isolated polynucleotide molecule comprising a nucleotide sequence selected from the group consisting of:
(a) a nucleotide sequence having at least 80% identity to a nucleotide sequence encoding an ADAMTS-SI polypeptide of SEQ ID NO: 2, or a metalloproteinase or disintegrin domain, prodomain, or thrombospondin (TSP) domain thereof;
(b) a nucleotide sequence of at least 15 contiguous nucleotides that hybridizes under stringent conditions to the polynucleotide molecule of SEQ ID NO: 1; and
(c) the complement of the nucleotide sequence of (a) or (b).

2. An isolated polynucleotide molecule of claim 1 wherein said polynucleotide sequence comprises the ADAMTS-SI polypeptide encoding sequence of SEQ ID NO: 2, or a sequence encoding a metalloproteinase or disintegrin domain, prodomain, or thrombospondin (TSP) domain thereof.

3. A polypeptide encoded by the isolated polynucleotide molecule of claim 1.

4. The polypeptide of claim 3 which comprises an amino acid sequence that is SEQ ID NO: 2 or a metalloproteinase or disintegrin domain, prodomain, or thrombospondin (TSP) domain thereof.

5. An expression system comprising a DNA or RNA molecule, wherein said expression system is capable of producing an ADAMTS-SI polypeptide of claim 3 when said expression system is present in a compatible host cell.

6. A host cell comprising the expression system of claim 5.

7. A process for producing an ADAMTS-SI polypeptide comprising culturing a host cell of claim 6 under conditions sufficient for production of said polypeptide, and recovering the polypeptide from cell culture.

8. An agent selected from the group consisting of an antibody immunospecific for an ADAMTS-SI polypeptide, an agonist for an ADAMTS-SI polypeptide, an antagonist for an ADAMTS-SI polypeptide, and a substrate for an ADAMTS-SI polypeptide, wherein said polypeptide is the polypeptide of claim 3.

9. Use of an agent of claim 8 in the manufacture of a medicament for treating a subject in need of altering activity or expression of ADAMTS-SI.

10. A process for diagnosing a disease or a susceptibility to a disease in a subject related to expression or activity of ADAMTS-SI in a subject comprising determining presence or absence of a mutation in a nucleotide sequence encoding a polypeptide of claim 3 in the genome of said subject, or analyzing for presence or amount of ADAMTS-SI expression in a sample derived from said subject.

11. A method for identifying compounds which antagonize, agonize, or bind to ADAMTS-SI comprising:
(a) contacting a candidate compound with cells expressing an ADAMTS-SI polypeptide of claim 3, or with cell membranes from cells expressing said ADAMTS-SI polypeptide, or the media conditioned by cells expressing said polypeptide, or a purified composition of said polypeptide; and
(b) determining inhibition or stimulation of an ADAMTS-SI activity, or binding or said candidate compound to said polypeptide.

12. A method for detecting a polynucleotide encoding ADAMTS-SI in a biological sample containing nucleic acid material comprising:
(a) hybridizing an isolated polynucleotide of claim 1 that is specific to ADAMTS-SI to the nucleic acid material of the biological sample, thereby forming a hybridization complex; and
(b) detecting the hybridization complex, wherein presence of the hybridization complex correlates with the presence of the polynucleotide encoding ADAMTS-SI in the biological sample.

13. A method for identifying a substrate for ADAMTS-SI comprising contacting a polypeptide comprising an enzymatically active polypeptide of claim 3 with a candidate substrate and determining either conversion of substrate to product or binding of the polypeptide to the substrate.

14. Use of an agent selected from the group consisting of an agonist or antagonist of ADAMTS-SI, a polypeptide of claim 3, and a polynucleotide of claim 1, in the manufacture of a medicament for treating arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock.

15. A pharmaceutical composition for the treatment of arthritis (osteoarthritis and rheumatoid arthritis), inflammatory bowel disease, Crohn's disease, emphysema, acute respiratory distress syndrome, asthma, chronic obstructive pulmonary disease, Alzheimer's disease, organ transplant toxicity and rejection, cachexia, allergy, cancer (such as solid tumor cancer including colon, breast, lung, prostate, brain and hematopoietic malignancies including leukemia and lymphoma), tissue ulcerations, restenosis, periodontal disease, epidermolysis bullosa, osteoporosis, loosening of artificial joints implants, atherosclerosis (including atherosclerotic plaque rupture), aortic aneurysm (including abdominal aortic and brain aortic aneurysm), congestive heart failure, myocardial infarction, stroke, cerebral ischemia, head trauma, spinal cord injury, neurodegenerative diseases (acute and chronic), autoimmune disorders, Huntington's disease, Parkinson's disease, migraine, depression, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, infertility or diabetic shock comprising a therapeutically effective amount of an agent selected from the group consisting of an agonist or antagonist of ADAMTS-SI, a polypeptide of claim 3, and a polynucleotide of claim 1, in combination with a pharmaceutically acceptable carrier.
